# EUROPEAN PATENT SPECIFICATION

(11) **EP 2 121 142 B1**
(45) Date of publication and mention of the grant of the patent: **03.04.2013**
(21) Application number: 08707263.3
(22) Date of filing: 24.01.2008
(51) Int. Cl.: A61K 8/60, A61K 8/64, A61Q 19/00, A61Q 19/08, C07K 14/51

(54) **USE OF GDF-5 FOR THE IMPROVEMENT OR MAINTENANCE OF DERMAL APPEARANCE**
VERWENDUNG VON GDF-5 ZUR VERBESSERUNG ODER AUFRECHTERHALTUNG DES AUSSEHENS DER HAUT
UTILISATION DE GDF-R POUR L'AMÉLIORATION OU L'ENTRETIEN DE L'ASPECT DERMIQUE

(30) Priority: 25.01.2007 EP 07001658
(43) Date of publication of application: 25.11.2009
(73) Proprietor: Biopharm Gesellschaft Zur Biotechnologischen Entwicklung Von Pharmaka mbH, 69115 Heidelberg (DE)
(72) Inventor: POHL, Jens, 76707 Hambrücken (DE)
(74) Representative: Böhm, Brigitte
(86) International application number: PCT/EP2008/000552
(87) International publication number: WO 2008/089987

(56) References cited:
- EP-A- 1 880 731
- WO-A-02/076494
- JENNER J M G TH ET AL: "Effect of transforming growth factor-beta and growth differentiation factor-5 on proliferation and matrix production by human bone marrow stromal cells cultured on braided poly lactic-co-glycolic acid scaffolds for ligament tissue engineering" TISSUE ENGINEERING, vol. 13, no. 7, July 2007 (2007-07), pages 1573-1582, XP002479562 ISSN: 1076-3279
- BATTAGLIA ET AL: "GDF-5 deficiency alters stress-relaxation properties in mouse skin" JOURNAL OF DERMATOLOGICAL SCIENCE, ELSEVIER SCIENCE PUBLISHERS, SHANNON, IR, vol. 39, no. 3, 1 September 2005 (2005-09-01), pages 192-195, XP005054252 ISSN: 0923-1811

### SEQUENCE LISTING

<110> Biopharm Gesellschaft zur biotechnologischen Entwicklung von Pharmaka mbH
<120> MP52 ECM
<130> 39708P EP
<160> 7
<170> PatentIn version 3.3
<210> 1
   <211> 501
   <212> PRT
   <213> homo sapiens
<220>
   <221> MISC_FEATURE
   <223> GDF-5 precursor
<400> 1
   Met Arg Leu Pro Lys Leu Leu Thr Phe Leu Leu Trp Tyr Leu Ala Trp
   1               5                   10                  15
   Leu Asp Leu Glu Phe Ile Cys Thr Val Leu Gly Ala Pro Asp Leu Gly
               20                  25                  30
   Gln Arg Pro Gln Gly Thr Arg Pro Gly Leu Ala Lys Ala Glu Ala Lys
           35                  40                  45
   Glu Arg Pro Pro Leu Ala Arg Asn Val Phe Arg Pro Gly Gly His Ser
       50                  55                  60
   Tyr Gly Gly Gly Ala Thr Asn Ala Asn Ala Arg Ala Lys Gly Gly Thr
   65                  70                  75                  80
   Gly Gln Thr Gly Gly Leu Thr Gln Pro Lys Lys Asp Glu Pro Lys Lys
                   85                  90                  95
   Leu Pro Pro Arg Pro Gly Gly Pro Glu Pro Lys Pro Gly His Pro Pro
               100                 105                 110
   Gln Thr Arg Gln Ala Thr Ala Arg Thr Val Thr Pro Lys Gly Gln Leu
           115                 120                 125
   Pro Gly Gly Lys Ala Pro Pro Lys Ala Gly Ser Val Pro Ser Ser Phe
       130                 135                 140
   Leu Leu Lys Lys Ala Arg Glu Pro Gly Pro Pro Arg Glu Pro Lys Glu
   145                 150                 155                 160
   Pro Phe Arg Pro Pro Pro Ile Thr Pro His Glu Tyr Met Leu Ser Leu
   165 170 175
   Tyr Arg Thr Leu Ser Asp Ala Asp Arg Lys Gly Gly Asn Ser Ser Val
   180 185 190
   Lys Leu Glu Ala Gly Leu Ala Asn Thr Ile Thr Ser Phe Ile Asp Lys
   195 200 205
   Gly Gln Asp Asp Arg Gly Pro Val Val Arg Lys Gln Arg Tyr Val Phe
   210 215 220
   Asp Ile Ser Ala Leu Glu Lys Asp Gly Leu Leu Gly Ala Glu Leu Arg
   225 230 235 240
   Ile Leu Arg Lys Lys Pro Ser Asp Thr Ala Lys Pro Ala Ala Pro Gly
   245 250 255
   Gly Gly Arg Ala Ala Gln Leu Lys Leu Ser Ser Cys Pro Ser Gly Arg
   260 265 270
   Gln Pro Ala Ser Leu Leu Asp Val Arg Ser Val Pro Gly Leu Asp Gly
   275 280 285
   Ser Gly Trp Glu Val Phe Asp Ile Trp Lys Leu Phe Arg Asn Phe Lys
   290 295 300
   Asn Ser Ala Gln Leu Cys Leu Glu Leu Glu Ala Trp Glu Arg Gly Arg
   305 310 315 320
   Ala Val Asp Leu Arg Gly Leu Gly Phe Asp Arg Ala Ala Arg Gln Val
   325 330 335
   His Glu Lys Ala Leu Phe Leu Val Phe Gly Arg Thr Lys Lys Arg Asp
   340 345 350
   Leu Phe Phe Asn Glu Ile Lys Ala Arg Ser Gly Gln Asp Asp Lys Thr
   355 360 365
   Val Tyr Glu Tyr Leu Phe Ser Gln Arg Arg Lys Arg Arg Ala Pro Leu
   370 375 380
   Ala Thr Arg Gln Gly Lys Arg Pro Ser Lys Asn Leu Lys Ala Arg Cys
   385 390 395 400
   Ser Arg Lys Ala Leu His Val Asn Phe Lys Asp Met Gly Trp Asp Asp
   405 410 415
   Trp Ile Ile Ala Pro Leu Glu Tyr Glu Ala Phe His Cys Glu Gly Leu
   420 425 430
   Cys Glu Phe Pro Leu Arg Ser His Leu Glu Pro Thr Asn His Ala Val
           435                 440                 445
   Ile Gln Thr Leu Met Asn Ser Met Asp Pro Glu Ser Thr Pro Pro Thr
       450                 455                 460
   Cys Cys Val Pro Thr Arg Leu Ser Pro Ile Ser Ile Leu Phe Ile Asp
   465                 470                 475                 480
   Ser Ala Asn Asn Val Val Tyr Lys Gln Tyr Glu Asp Met Val Val Glu
                   485                 490                 495
   Ser Cys Gly Cys Arg
               500
<210> 2
   <211> 2703
   <212> DNA
   <213> homo sapiens
<220>
   <221> CDS
   <222> (640)..(2142)
<400> 2
   ccatggcctc gaaagggcag cggtgatttt tttcacataa atatatcgca cttaaatgag     60
   tttagacagc atgacatcag agagtaatta aattggtttg ggttggaatt ccgtttccaa    120
   ttcctgagtt caggtttgta aaagattttt ctgagcacct gcaggcctgt gagtgtgtgt    180
   gtgtgtgtgt gtgtgtgtgt gtgtgtgtga agtattttca ctggaaagga ttcaaaacta    240
   gggggaaaaa aaaactggag cacacaggca gcattacgcc attcttcctt cttggaaaaa    300
   tccctcagcc ttatacaagc ctccttcaag ccctcagtca gttgtgcagg agaaaggggg    360
   cggttggctt tctcctttca agaacgagtt attttcagct gctgactgga gacggtgcac    420
   gtctggatac gagagcattt ccactatggg actggataca aacacacacc cggcagactt    480
   caagagtctc agactgagga gaaagccttt ccttctgctg ctactgctgc tgccgctgct    540
   tttgaaagtc cactcctttc atggtttttc ctgccaaacc agaggcacct ttgctgctgc    600
   cgctgttctc tttggtgtca ttcagcggct ggccagagg atg aga ctc ccc aaa       654
                                             Met Arg Leu Pro Lys
                                             1               5
   ctc ctc act ttc ttg ctt tgg tac ctg gct tgg ctg gac ctg gaa ttc      702
   Leu Leu Thr Phe Leu Leu Trp Tyr Leu Ala Trp Leu Asp Leu Glu Phe
                   10                  15                  20
   atc tgc act gtg ttg ggt gcc cct gac ttg ggc cag aga ccc cag ggg      750
   Ile Cys Thr Val Leu Gly Ala Pro Asp Leu Gly Gln Arg Pro Gln Gly
               25                  30                  35
   acc agg cca gga ttg gcc aaa gca gag gcc aag gag agg ccc ccc ctg      798
   Thr Arg Pro Gly Leu Ala Lys Ala Glu Ala Lys Glu Arg Pro Pro Leu
           40                      45                      50
   gcc cgg aac gtc ttc agg cca ggg ggt cac agc tat ggt ggg ggg gcc       846
   Ala Arg Asn Val Phe Arg Pro Gly Gly His Ser Tyr Gly Gly Gly Ala
       55                  60                  65
   acc aat gcc aat gcc agg gca aag gga ggc acc ggg cag aca gga ggc       894
   Thr Asn Ala Asn Ala Arg Ala Lys Gly Gly Thr Gly Gln Thr Gly Gly
   70                  75                  80                  85
   ctg aca cag ccc aag aag gat gaa ccc aaa aag ctg ccc ccc aga ccg       942
   Leu Thr Gln Pro Lys Lys Asp Glu Pro Lys Lys Leu Pro Pro Arg Pro
                   90                  95                  100
   ggc ggc cct gaa ccc aag cca gga cac cct ccc caa aca agg cag gct       990
   Gly Gly Pro Glu Pro Lys Pro Gly His Pro Pro Gln Thr Arg Gln Ala
               105                 110                 115
   aca gcc cgg act gtg acc cca aaa gga cag ctt ccc gga ggc aag gca      1038
   Thr Ala Arg Thr Val Thr Pro Lys Gly Gln Leu Pro Gly Gly Lys Ala
           120                 125                 130
   ccc cca aaa gca gga tct gtc ccc agc tcc ttc ctg ctg aag aag gcc      1086
   Pro Pro Lys Ala Gly Ser Val Pro Ser Ser Phe Leu Leu Lys Lys Ala
       135                 140                 145
   agg gag ccc ggg ccc cca cga gag ccc aag gag ccg ttt cgc cca ccc      1134
   Arg Glu Pro Gly Pro Pro Arg Glu Pro Lys Glu Pro Phe Arg Pro Pro
   150                 155                 160                 165
   ccc atc aca ccc cac gag tac atg ctc tcg ctg tac agg acg ctg tcc      1182
   Pro Ile Thr Pro His Glu Tyr Met Leu Ser Leu Tyr Arg Thr Leu Ser
                   170                 175                 180
   gat gct gac aga aag gga ggc aac agc agc gtg aag ttg gag gct ggc      1230
   Asp Ala Asp Arg Lys Gly Gly Asn Ser Ser Val Lys Leu Glu Ala Gly
               185                 190                 195
   ctg gcc aac acc atc acc agc ttt att gac aaa ggg caa gat gac cga      1278
   Leu Ala Asn Thr Ile Thr Ser Phe Ile Asp Lys Gly Gln Asp Asp Arg
           200                 205                 210
   ggt ccc gtg gtc agg aag cag agg tac gtg ttt gac att agt gcc ctg      1326
   Gly Pro Val Val Arg Lys Gln Arg Tyr Val Phe Asp Ile Ser Ala Leu
       215                 220                 225
   gag aag gat ggg ctg ctg ggg gcc gag ctg cgg atc ttg cgg aag aag      1374
   Glu Lys Asp Gly Leu Leu Gly Ala Glu Leu Arg Ile Leu Arg Lys Lys
   230                 235                 240                 245
   ccc tcg gac acg gcc aag cca gcg gcc ccc gga ggc ggg cgg gct gcc      1422
   Pro Ser Asp Thr Ala Lys Pro Ala Ala Pro Gly Gly Gly Arg Ala Ala
                   250                 255                 260
   cag ctg aag ctg tcc agc tgc ccc agc ggc cgg cag ccg gcc tcc ttg      1470
   Gln Leu Lys Leu Ser Ser Cys Pro Ser Gly Arg Gln Pro Ala Ser Leu
               265                 270                 275
   ctg gat gtg cgc tcc gtg cca ggc ctg gac gga tct ggc tgg gag gtg      1518
   Leu Asp Val Arg Ser Val Pro Gly Leu Asp Gly Ser Gly Trp Glu Val
           280                 285                 290
   ttc gac atc tgg aag ctc ttc cga aac ttt aag aac tcg gcc cag ctg      1566
   Phe Asp Ile Trp Lys Leu Phe Arg Asn Phe Lys Asn Ser Ala Gln Leu
       295                 300                 305
   tgc ctg gag ctg gag gcc tgg gaa cgg ggc agg gcc gtg gac ctc cgt      1614
   Cys Leu Glu Leu Glu Ala Trp Glu Arg Gly Arg Ala Val Asp Leu Arg
   310                 315                 320                 325
   ggc ctg ggc ttc gac cgc gcc gcc cgg cag gtc cac gag aag gcc ctg      1662
   Gly Leu Gly Phe Asp Arg Ala Ala Arg Gln Val His Glu Lys Ala Leu
                   330                 335                 340
   ttc ctg gtg ttt ggc cgc acc aag aaa cgg gac ctg ttc ttt aat gag      1710
   Phe Leu Val Phe Gly Arg Thr Lys Lys Arg Asp Leu Phe Phe Asn Glu
               345                 350                 355
   att aag gcc cgc tct ggc cag gac gat aag acc gtg tat gag tac ctg      1758
   Ile Lys Ala Arg Ser Gly Gln Asp Asp Lys Thr Val Tyr Glu Tyr Leu
           360                 365                 370
   ttc agc cag cgg cga aaa cgg cgg gcc cca ctg gcc act cgc cag ggc      1806
   Phe Ser Gln Arg Arg Lys Arg Arg Ala Pro Leu Ala Thr Arg Gln Gly
       375                 380                 385
   aag cga ccc agc aag aac ctt aag gct cgc tgc agt cgg aag gca ctg      1854
   Lys Arg Pro Ser Lys Asn Leu Lys Ala Arg Cys Ser Arg Lys Ala Leu
   390                 395                 400                 405
   cat gtc aac ttc aag gac atg ggc tgg gac gac tgg atc atc gca ccc      1902
   His Val Asn Phe Lys Asp Met Gly Trp Asp Asp Trp Ile Ile Ala Pro
                   410                 415                 420
   ctt gag tac gag gct ttc cac tgc gag ggg ctg tgc gag ttc cca ttg      1950
   Leu Glu Tyr Glu Ala Phe His Cys Glu Gly Leu Cys Glu Phe Pro Leu
               425                 430                 435
   cgc tcc cac ctg gag ccc acg aat cat gca gtc atc cag acc ctg atg      1998
   Arg Ser His Leu Glu Pro Thr Asn His Ala Val Ile Gln Thr Leu Met
           440                 445                 450
   aac tcc atg gac ccc gag tcc aca cca ccc acc tgc tgt gtg ccc acg      2046
   Asn Ser Met Asp Pro Glu Ser Thr Pro Pro Thr Cys Cys Val Pro Thr
       455                 460                 465
   cgg ctg agt ccc atc agc atc ctc ttc att gac tct gcc aac aac gtg      2094
   Arg Leu Ser Pro Ile Ser Ile Leu Phe Ile Asp Ser Ala Asn Asn Val
   470                 475                 480                 485
   gtg tat aag cag tat gag gac atg gtc gtg gag tcg tgt ggc tgc agg      2142
   Val Tyr Lys Gln Tyr Glu Asp Met Val Val Glu Ser Cys Gly Cys Arg
                   490                 495                 500
   tagcagcact ggccctctgt cttcctgggt ggcacatccc aagagcccct tcctgcactc    2202
   ctggaatcac agaggggtca ggaagctgtg gcaggagcat ctacacagct tgggtgaaag    2262
   gggattccaa taagcttgct cgctctctga gtgtgacttg ggctaaaggc ccccttttat    2322
   ccacaagttc ccctggctga ggattgctgc ccgtctgctg atgtgaccag tggcaggcac    2382
   aggtccaggg agacagactc tgaatgggac tgagtcccag gaaacagtgc tttccgatga    2442
   gactcagccc accatttctc ctcacctggg ccttctcagc ctctggactc tcctaagcac    2502
   ctctcaggag agccacaggt gccactgcct cctcaaatca catttgtgcc tggtgacttc    2562
   ctgtccctgg gacagttgag aagctgactg ggcaagagtg ggagagaaga ggagagggct    2622
   tggatagagt tgaggagtgt gaggctgtta gactgttaga tttaaatgta tattgatgag    2682
   ataaaaagca aaactgtgcc t 2703
<210> 3
   <211> 120
   <212> PRT
   <213> Homo sapiens
<220>
   <223> aa sequence of human GDF-5; the protein my alternatively consist of 119 aa, thus beginning with proline
<400> 3
   Ala Pro Leu Ala Thr Arg Gln Gly Lys Arg Pro Ser Lys Asn Leu Lys
   1               5                   10                  15
   Ala Arg Cys Ser Arg Lys Ala Leu His Val Asn Phe Lys Asp Met Gly
               20                  25                  30
   Trp Asp Asp Trp Ile Ile Ala Pro Leu Glu Tyr Glu Ala Phe His Cys
           35                  40                  45
   Glu Gly Leu Cys Glu Phe Pro Leu Arg Ser His Leu Glu Pro Thr Asn
       50                  55                  60
   His Ala Val Ile Gln Thr Leu Met Asn Ser Met Asp Pro Glu Ser Thr
   65                  70                  75                  80
   Pro Pro Thr Cys Cys Val Pro Thr Arg Leu Ser Pro Ile Ser Ile Leu
                   85                  90                  95
   Phe Ile Asp Ser Ala Asn Asn Val Val Tyr Lys Gln Tyr Glu Asp Met
               100                 105                 110
   Val Val Glu Ser Cys Gly Cys Arg
           115                 120
<210> 4
   <211> 120
   <212> PRT
   <213> Homo sapiens
<220>
   <223> aa sequence of monomeric human GDF-5; the protein my alternatively consist of 119 aa, thus beginning with proline;
<400> 4
   Ala Pro Leu Ala Thr Arg Gln Gly Lys Arg Pro Ser Lys Asn Leu Lys
   1               5                   10                  15
   Ala Arg Cys Ser Arg Lys Ala Leu His Val Asn Phe Lys Asp Met Gly
               20                  25                  30
   Trp Asp Asp Trp Ile Ile Ala Pro Leu Glu Tyr Glu Ala Phe His Cys
           35                  40                  45
   Glu Gly Leu Cys Glu Phe Pro Leu Arg Ser His Leu Glu Pro Thr Asn
       50                  55                  60
   His Ala Val Ile Gln Thr Leu Met Asn Ser Met Asp Pro Glu Ser Thr
   65                  70                  75                  80
   Pro Pro Thr Ala Cys Val Pro Thr Arg Leu Ser Pro Ile Ser Ile Leu
   85 90 95
   Phe Ile Asp Ser Ala Asn Asn Val Val Tyr Lys Gln Tyr Glu Asp Met
   100 105 110
   Val Val Glu Ser Cys Gly Cys Arg
   115 120
<210> 5
   <211> 102
   <212> PRT
   <213> homo sapiens
<220>
   <221> MISC_FEATURE
   <223> cystine-knot domain of GDF-6
<400> 5
   Cys Ser Lys Lys Pro Leu His Val Asn Phe Lys Glu Leu Gly Trp Asp
   1 5 10 15
   Asp Trp Ile Ile Ala Pro Leu Glu Tyr Glu Ala Tyr His Cys Glu Gly
   20 25 30
   Val Cys Asp Phe Pro Leu Arg Ser His Leu Glu Pro Thr Asn His Ala
   35 40 45
   Ile Ile Gln Thr Leu Met Asn Ser Met Asp Pro Gly Ser Thr Pro Pro
   50 55 60
   Ser Cys Cys Val Pro Thr Lys Leu Thr Pro Ile Ser Ile Leu Tyr Ile
   65 70 75 80
   Asp Ala Gly Asn Asn Val Val Tyr Lys Gln Tyr Glu Asp Met Val Val
   85 90 95
   Glu Ser Cys Gly Cys Arg
   100
<210> 6
   <211> 102
   <212> PRT
   <213> homo sapiens
<220>
   <221> MISC_FEATURE
   <223> cystine-knot domain of GDF-7
<400> 6
   Cys Ser Arg Lys Pro Leu His Val Asp Phe Lys Glu Leu Gly Trp Asp
   1 5 10 15
   Asp Trp Ile Ile Ala Pro Leu Asp Tyr Glu Ala Tyr His Cys Glu Gly
   20 25 30
   Leu Cys Asp Phe Pro Leu Arg Ser His Leu Glu Pro Thr Asn His Ala
   35 40 45
   Ile Ile Gln Thr Leu Leu Asn Ser Met Ala Pro Asp Ala Ala Pro Ala
   50 55 60
   Ser Cys Cys Val Pro Ala Arg Leu Ser Pro Ile Ser Ile Leu Tyr Ile
   65 70 75 80
   Asp Ala Ala Asn Asn Val Val Tyr Lys Gln Tyr Glu Asp Met Val Val
   85 90 95
   Glu Ala Cys Gly Cys Arg
   100
<210> 7
   <211> 102
   <212> PRT
   <213> homo sapiens
<220>
   <221> MISC_FEATURE
   <223> cystine-knot domain of GDF-5
<400> 7
   Cys Ser Arg Lys Ala Leu His Val Asn Phe Lys Asp Met Gly Trp Asp
   1 5 10 15
   Asp Trp Ile Ile Ala Pro Leu Glu Tyr Glu Ala Phe His Cys Glu Gly
   20 25 30
   Leu Cys Glu Phe Pro Leu Arg Ser His Leu Glu Pro Thr Asn His Ala
   35 40 45
   Val Ile Gln Thr Leu Met Asn Ser Met Asp Pro Glu Ser Thr Pro Pro
   50 55 60
   Thr Cys Cys Val Pro Thr Arg Leu Ser Pro Ile Ser Ile Leu Phe Ile
   65 70 75 80
   Asp Ser Ala Asn Asn Val Val Tyr Lys Gln Tyr Glu Asp Met Val Val
   85 90 95
   Glu Ser Cys Gly Cys Arg
   100

## Description

The present invention is directed to novel cosmetic methods and uses of compositions containing growth and differentiation factor proteins. Said compositions are specifically designed to optimize the amount of crucial structural elements of mammalian tissues for the preservation and improvement of dermal tissue appearance.

The reduction of structural proteins such as collagen in various tissues is a frequently occurring age- and/or environmental-related problem. In the skin, such gradual or sudden changes are typically accompanied by a decline of elasticity, increase of facial wrinkles and liver spots, loss of skin gloss and other undesired alterations. The process is influenced by several factors including genetics, UV irradiation, xenobiotics, mechanical stress, hormonal changes, and metabolic processes (creation of reactive substances such as radicals, sugars, and aldehydes). Taken together, all these factors lead to cumulative alterations of skin structure, function, and appearance. As a result, skin ages.

The influence of the environment, especially solar UV irradiation, is of considerable importance for dermal aging. Photoaged skin appears wrinkled, lax, coarse, with uneven pigmentation, and brown spots. Histological and ultrastructural studies have revealed that photodamaged skin is associated with increased epidermal thickness, impairment of the fibrillar organization of collagen and elastin, and alterations of connective tissue organization. Skin aging due to UV exposure is superimposed on chronological skin aging. Historically, photoaging and chronological skin aging have been considered to be distinct entities. However, recent evidence indicates that chronologically aged and UV-irradiated skin share important molecular features including altered signal transduction pathways that promote matrix-metalloproteinase (MMP) expression, enhanced degradation and decreased synthesis of structural proteins, and connective tissue damage. This concordance of molecular mechanisms suggests that UV irradiation accelerates many key aspects of the chronological aging process in human skin. Even in individuals who are not exposed to exceedingly deleterious environmental factors, the amount of collagen and other scaffold substances in the skin tends to decline with age. For example, a child's skin is rich of collagen III which is partly responsible for the softness of the young skin. As the body growth slows down, the skin content of type III collagen declines, while that of type I increases for another couple of years. Type I collagen continues to build up until about the age of 35, when the skin reaches the peak of its mechanical strength. By the age of 60, all types of structural proteins such as collagen, elastin, fibrillin and decorin are significantly below their youthful levels. Thus, it is commonly accepted that a remarkable reduction of the level of protein fibres in the dermis is a major causative of skin aging.

The use of natural or synthetic cosmetics to treat the appearance of the face and condition of the skin is common among many cultures. In the past, various dermal renewal methods have been developed but all have drawbacks. Cosmetics supplemented with collagen and mucopolysaccharides such as hyaluronic add for imparting humectancy to the skin have been conventionally explored to prevent the skin aging. Unfortunately they could not yet attain a sufficient effect on such purpose. Since collagen and other structural proteins are not substantially absorbed if extradermally applied to the skin, these compositions could not augment the activity of fibroblasts and keratinocytes. Retinoic acid as an additive slowly remodels skin but at the price of chronic irritation and redness. Melatonin and vitamin C both increase skin collagen but skin also needs to increase its rebuild of elastin and water-holding proteoglycans. Controlled skin damage (i.e. peels, dermabrasion, lasers) works well only if there is a vigorous post-therapy regenerative response by the damaged skin.

In order to prevent skin aging and/or to maintain/promote health, it is therefore desired to explore agents which are able of augmenting the production of collagen and other main constituents for dermis, tissues, and organs. The most recent developments of cosmetic compositions (i.e. described in patent application US2002081324) utilize selected growth factor proteins such as platelet derived growth factor (PDGF), epidermal growth factor (EGF), and insulin-like growth factors (IGF-I and IGF-II), which have been effective in stimulating skin cell renewal. Products containing recombinant N-furfuryladenine (kinetin) plant growth factor, epidermal growth factor (ReVive Skincare) and recombinant TGF-ß (CRS, Topix Pharmaceuticals, NY, USA) are already marketed.

Unlike previously disclosed compositions for the aforementioned purposes, the cosmetic methods and uses of the present invention relate to proteins which are identical with or very closely related to growth/differentiation factor-5 (GDF-5).

GDF-5 (Hötten et al. 1994, Biochem. Biophys Res. Commun. 204, 646-652) is a morphogen which has been shown to promote cell proliferation, differentiation and/or tissue formation in several tissues. The protein is also known as morphogenic protein MP52, bone morphogenetic protein BMP-14 or cartilage-derived morphogenetic protein-1 (CDMP-1). GDF-5 is closely related to GDF-6 and GDF-7. These three proteins form a distinct subgroup of the TGF-ß superfamily, thus displaying comparable biological properties and an extraordinary high degree of amino acid sequence identity (see i.e. Wolfman et al. 1997, J. Clin. Invest. 100, 321-330). It has repeatedly been demonstrated that members of the GDF-5/-6/-7 subgroup are primarily important inducers and regulators of bone and cartilage (Cheng et al. 2003, J. Bone & Joint Surg. 85A, 1544-1552; Settle et al. 2003, Developm. Biol. 254, 116-130) as well as tendon/ligament (Wolfman et al. 1997, J.. Clin. Invest 100, 321-330). All family members are initially synthesized as larger

precursor proteins which subsequently undergo proteolytic cleavage at a cluster of basic residues approximately 110-140 amino acids from the C-terminus, thus releasing the C-terminal mature protein parts from the N-terminal prodomain. The mature polypeptides are structurally related and contain a conserved bioactive domain comprising six or seven canonical cysteine residues which is responsible for the characteristical three dimensional "cystine-knot" motif of these proteins. Native GDF-5 related proteins are homodimeric molecules and act mainly through interaction with specific receptor complexes which are composed of type I and type II serine/threonine receptor kinases. The receptor kinases subsequently activate Smad proteins, which then propagate the signals into the nucleus to regulate target gene expression.

Although there have been moderate advances in the art, an effective treatment of an age- and/or environmental-related decline of structural cell components in the skin remains a big challenge. It is therefore an object of the present invention to provide novel cosmetic methods and uses aiming at the preservation and improvement of dermal tissue appearance.

### Definitions:

In order to avoid misunderstandings and ambiguities, some frequently used terms herein are defined and exemplified as follows:
The term "cystine-knot domain" as used herein means the well known and conserved cysteine-rich amino acid region which is present in the mature parts of TGF-beta superfamily proteins such as i.e. human GDF-5 and forms a three-dimensional protein structure known as cystine-knot. In this domain the respective location of the cysteine residues.to each other is important and is only allowed to vary slightly in order not to lose the biological activity.

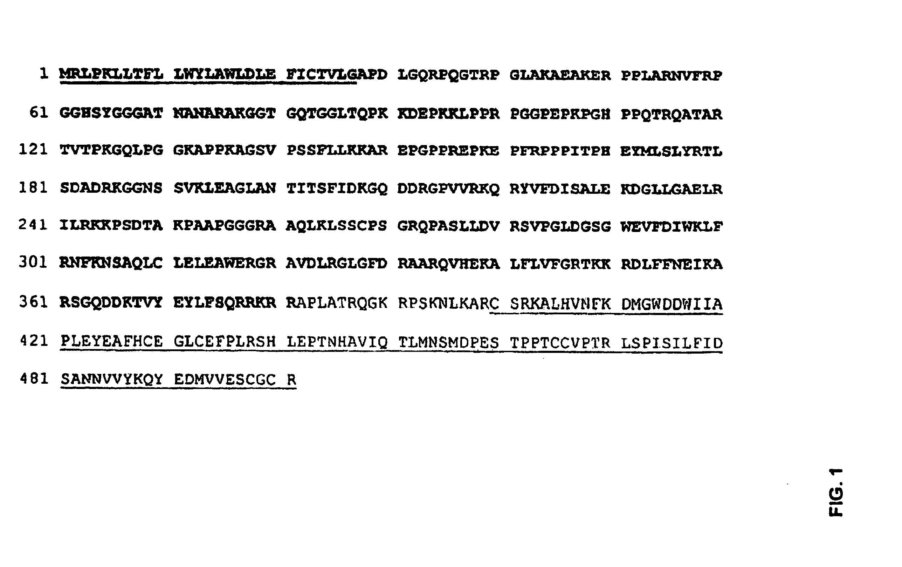

it has been demonstrated that the cystine-knot domain alone is sufficient for the biological function of the protein (Schreuder et al. (2005), Biochem Biophys Res Commun. 329, 1076-86). Consensus sequences for cystine-knot domains are well known in the state of the art. According to the definition defined herein the cystine-knot-domain of a protein starts with the first cysteine residue participating in the cystine-knot of the respective protein and ends with the residue which follows the last cysteine participating in the cystine-knot of the respective protein. For example, the cystine-knot domain of the human GDF-5 precursor protein (SEQ ID NO: 1) consists of the amino acids 400-501 (see also FIG. 1).

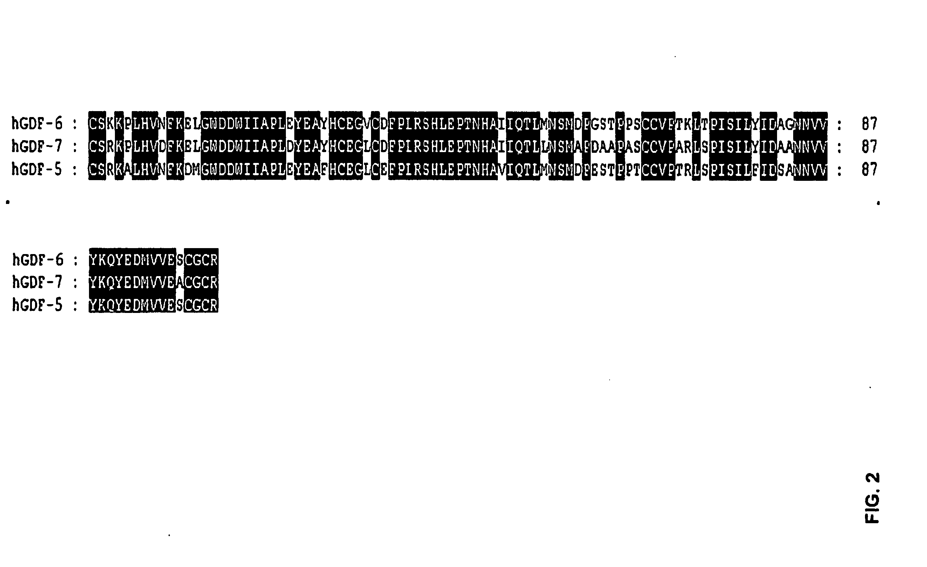

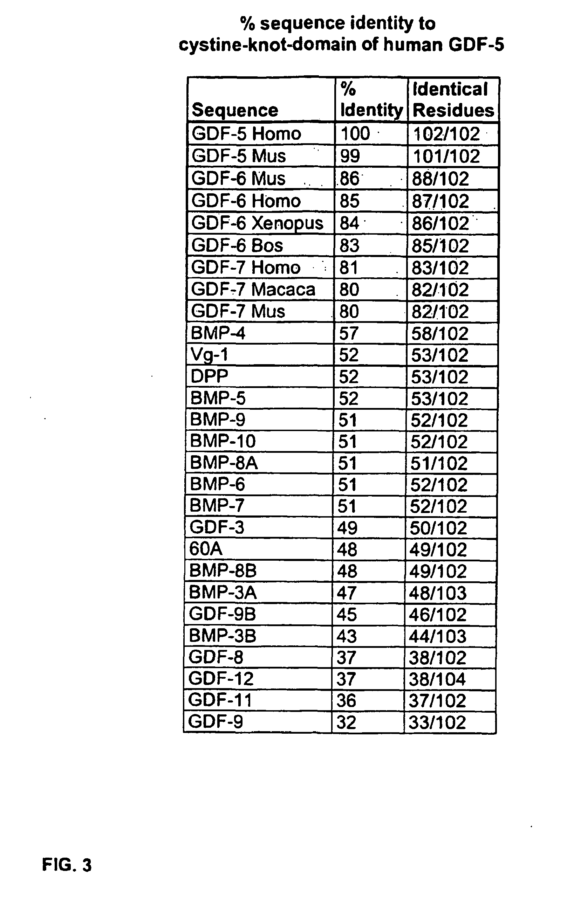

The term "GDF-5-related protein" as used herein means any naturally occurring or artificially created protein which comprises a cystine-knot-domain with an amino acid identity of at least 60% to the 102 aa cystine-knot domain of human GDF-5 (amino adds 400-501 of SEQ ID NO: 1). This term includes proteins belonging to the group of GDF-5, GDF-6 and GDF-7 proteins from vertebrate or mammalian species as well as recombinant variants thereof as long as these proteins show the above mentioned percentage of identity with the cystine-knot domain of human GDF-5. The limiting value of 60% is well suitable to separate members of the GDF-5/-6/-7 group of proteins as well as variants thereof from further proteins such as other GDFs and BMPs. A comparison of the 102 aa cystine-knot-domains of human GDF-5, human GDF-6 and human GDF-7 (see FIG. 2) reveals the high grade of amino acid identity between these proteins. Human GDF-6 shares 87 (85%) and human GDF-7 shares 83 (81%) identical residues with the cystine-knot-domain of human GDF-5. The respective domains of GDF-5/-6/-7 molecules from other vertebrate and mammalian species which have been identified so far also show very high identity percentages of at least 75% (between 79% and 99%), when compared with human GDF-5. In contrast, GDFs and BMPs not belonging to the GDF-5/-6/-7 subgroup display much lower identity values below 60% (see FIG. 3)

The determination of corresponding amino acid positions in related amino acid sequences as well as the calculation of percentages of identity between can be easily performed with the help of well known alignment algorithms and optionally computer programs using these algorithms. For example, the amino acid identities in this patent application (i.e. FIG. 2) have been calculated by aligning sequences with the freeware program ClustalX (Version 1.81) with default parameters and subsequent counting of identical residues by hand. Default settings for pairwise alignment (slow-accurate) are: gap opening parameter: 10.00; gap extension parameter 0.10; Protein weight matrix: Gonnet 250. The ClustalX program is described in detail in Thompson,J.D., Gibson,T.J., Plewniak,F., Jeanmougin,F. and Higgins,D.G. (1997): The ClustalX windows interface: flexible strategies for multiple sequence alignment aided by quality analysis tools. Nucleic Acids Research 24:4876-4882. ClustalX is a windows interface for the ClustalW multiple sequence alignment program and is i.e. available from various sources, i.e. by anonymous ftp from ftp-igbmc.u-strasbg.fr, ftp.embl-heidelberg.de, ftp.ebi.ac.uk or via download from the following webpage: http://www-igbmc.u-strasbg.fr/Biolnfo/. The ClustalW program and algorithm is also described in detail in Thompson, J.D., Higgins, D.G. and Gibson, T.J. (1994): CLUSTALW: improving the sensitivity of progressive multiple sequence alignment through sequence weighting, positions-specific gap penalties and weight matrix choice. Nucleic Acids Research 22:4673-4680.

The term "cosmetic composition" refers to non-medical compositions or formulations which are primarily designed to improve skin composition and/or function by the delivery of nutrients and/or other bioactive substances..The main object of cosmetic compositions is to alleviate dermal aging and to achieve a more appealing skin appearance. However, the term "cosmetic compositions" comprises "cosmeceuticals" - compositions which are sold as cosmetics but nevertheless include comparatively small amounts of pharmaceutically active substances (see Elsner, P., Maibach, H., editors. Cosmeceuticals and active cosmetics (2005). Boca Raton (FL): Taylor and Francis Group).

The terms "dermal", "dermal cells" and "dermal tissues" refer to cells and tissues which are present in mammalian skin.

The term "colloid" refers to small particles dispersed in a liquid.

The terms "lipid microparticle formulation" and "LMP formulation" refer to homogeneous oil/water microemulsions containing small spherical particles having a particle size below 200 nm.

The term "enhanced solubility" in context with GDF-5-related proteins refers to a stabilization of the GDF-5-related protein in blood and/or aqueous solutions at a pH between 4.0 and 9.0. This stabilization may be achieved by the creation of a stable liquid formulation or aqueous emulsion such as the colloidal formulations according to the invention.

The term "physiological pH" as used herein means pH within the range of 4 to 8.5 and more preferably within the range of 5 to 7.5.

The term "biological activity" denotes the activity of compounds, including, e.g., a GDF-5-related protein as measured by the common in vitro alkaline phosphatase assay (ALP), e.g. as described in example 2 or in Takuwa et al. (1989), Am. J. Physiol. 257, E797-E803). Suitable cell lines which may be used in such ALP assay are e.g. ATDC-5 or MCHT 1/26 cells.

It has now surprisingly been found that cosmetic methods which utilize GDF-5-related proteins can be efficiently used to stimulate the synthesis of structural elements of mammalian tissues.

Mammalian skin is the body's largest and ever changing organ that contains many specialized cells and structures. It is composed of three primary layers: the outer layer or epidermis, which provides waterproofing and serves as a barrier to infection; the dermis, which serves as a location for the appendages of skin; and the basement membrane, which separates the epidermis from the dermis.

The main cell type which makes up the epidermis is the keratinocyte. The epidermal skin layer contains no blood vessels; it is a natural barrier which is responsible to keep water in the body and protect against harmful chemicals and pathogens. The epidermis consists of five sublayers: stratum basale, stratum spinosum, stratum granulosum, stratum licidum and stratum corneum. In the bottom layer (stratum basale) the cells divide and push already formed cells into higher layers. The top layer of the epidermis, the stratum corneum, is made of dead, flat skin cells that shed about every two weeks.

The basement membrane is a thin but complex layer of tissue which joins the epidermis and dermis of the skin. This anchoring complex is composed of the hemidesmosomes, anchoring filaments, and anchoring fibrils. It contains several layers such as i.e. lamina lucida which comprise glycoproteins (e.g. laminin) and collagens.

The dermis is the inner layer of the skin and consists of loose connective tissue. Although it contains also a number of cellular structures including blood vessels, nerves, hair follicles, smooth muscle, glands and lymphatic tissue, the dermis is relatively cellular. A significant part of its volume is extracellular space, which is filled by an organized network of interwoven macromolecules called extracellular matrix (ECM).

Major components of the ECM of the dermis and of other tissues are proteoglycans (i.e. decorin), mucopolysaccarides (i.e. hyaluronic acid) and fibrous proteins such as collagens, elastin and fibrillin. The proteoglycan molecules form a highly hydrated, gel-like "ground substance" in which the fibrous proteins are embedded. Hyaluronic acid locks moisture and supports the structural integrity of the extracellular matrix. The collagen fibers both strengthen and help organize the matrix. Type I and type III collagens are the most common collagens which are i.e. present in the ECM of dermal tissues. Type IV collagen molecules assemble into a meshwork that constitutes a major part of the mature basal lamina, a thin mat of specialized extracellular matrix that underlie all epithelial cell sheets and tubes. The basal lamina is tethered to the underlying connective tissue by specialized anchoring fibrils made of type VII collagens. The term basement membrane is often used to describe the composite of the basal lamina and this layer of collagen fibrils. Rubberlike elastic fibers composed of elastin and microfibrillar glycoproteins (i.e. fibrillins -1 and -2) give the extracellular matrix resilience.

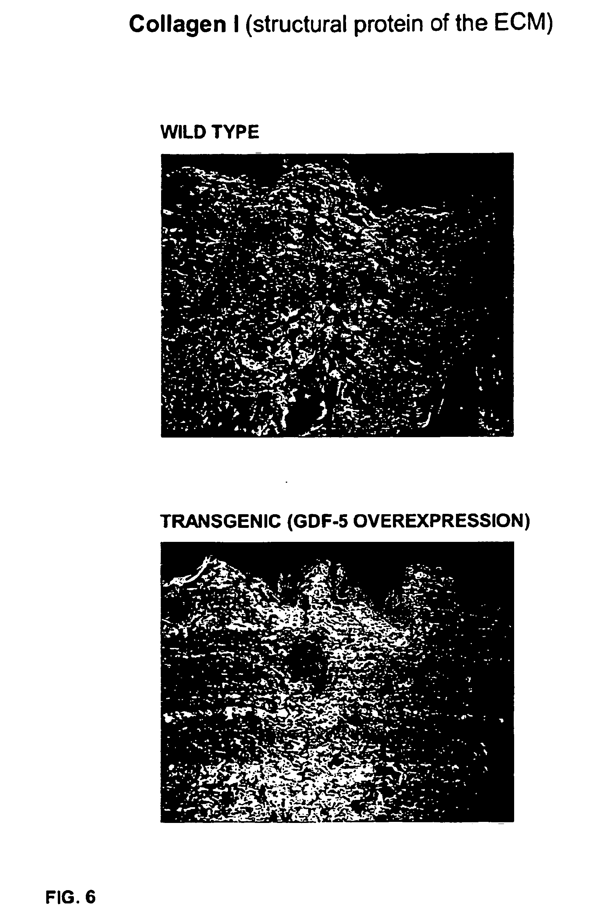

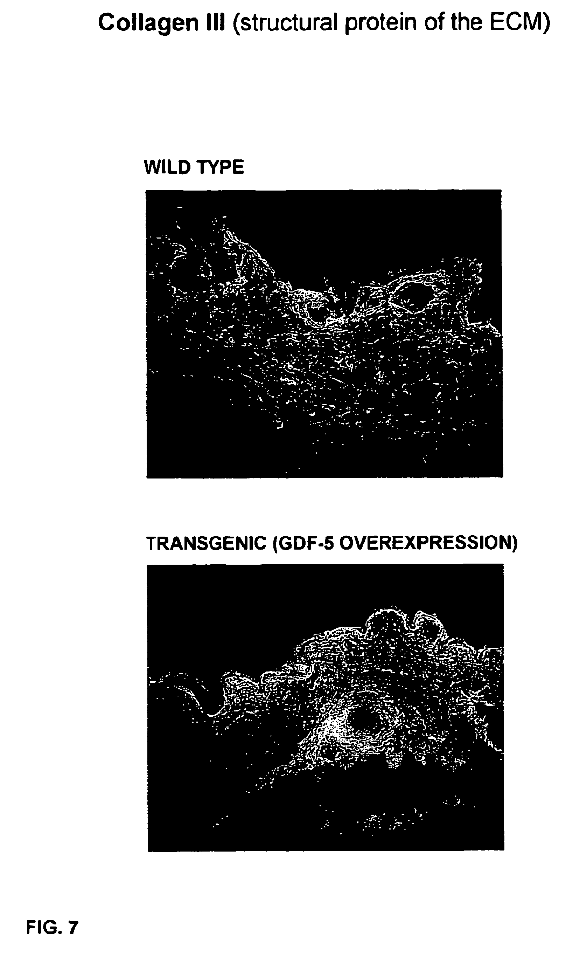

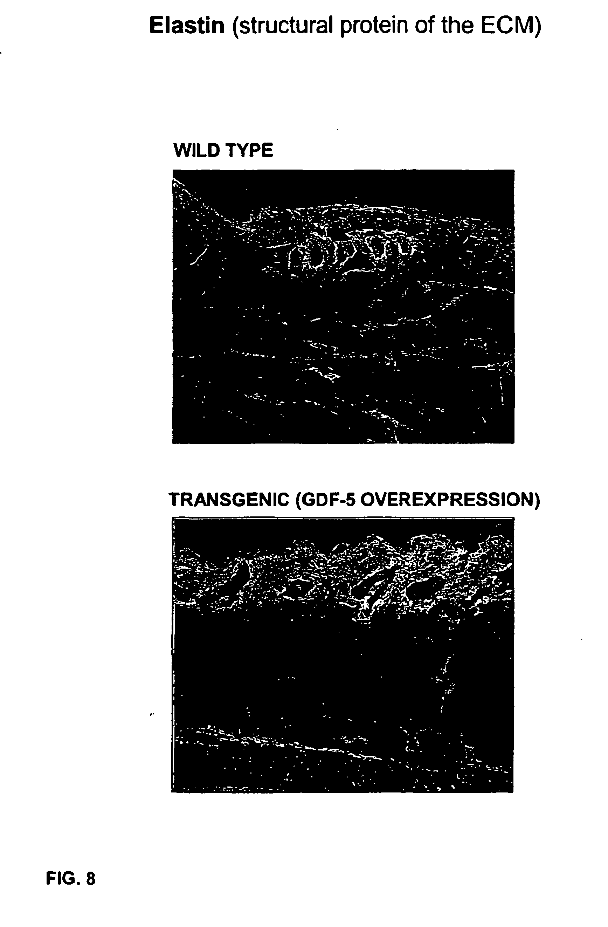

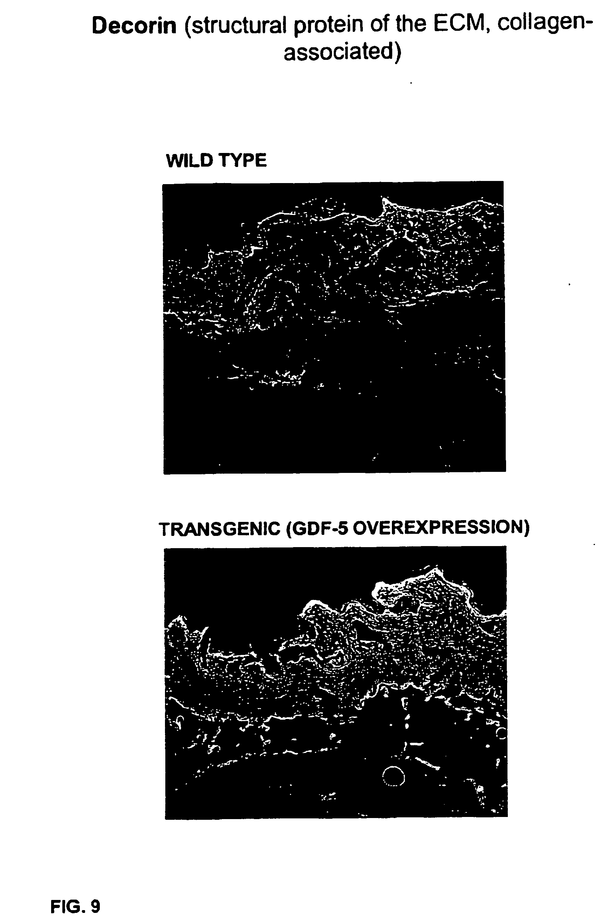

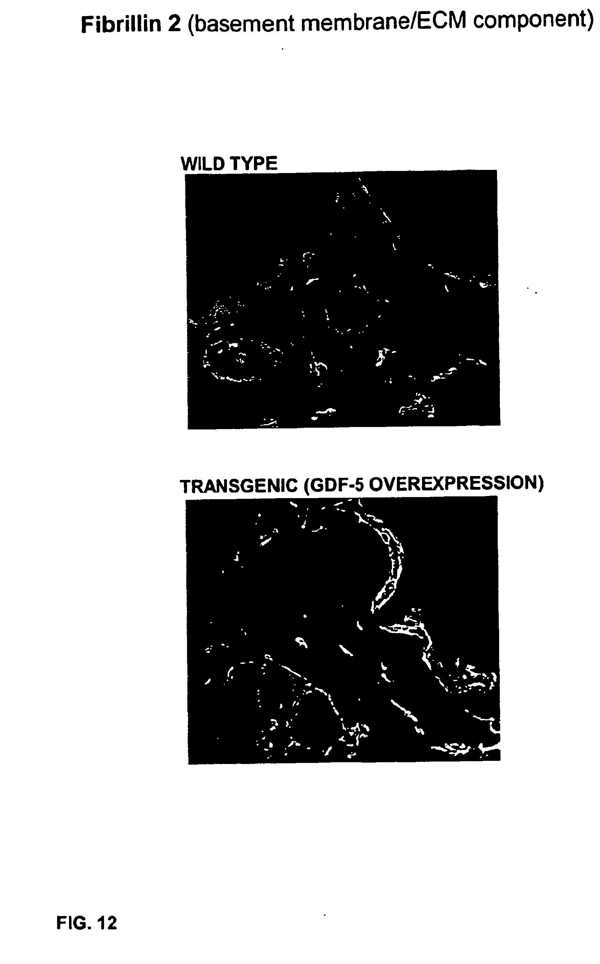

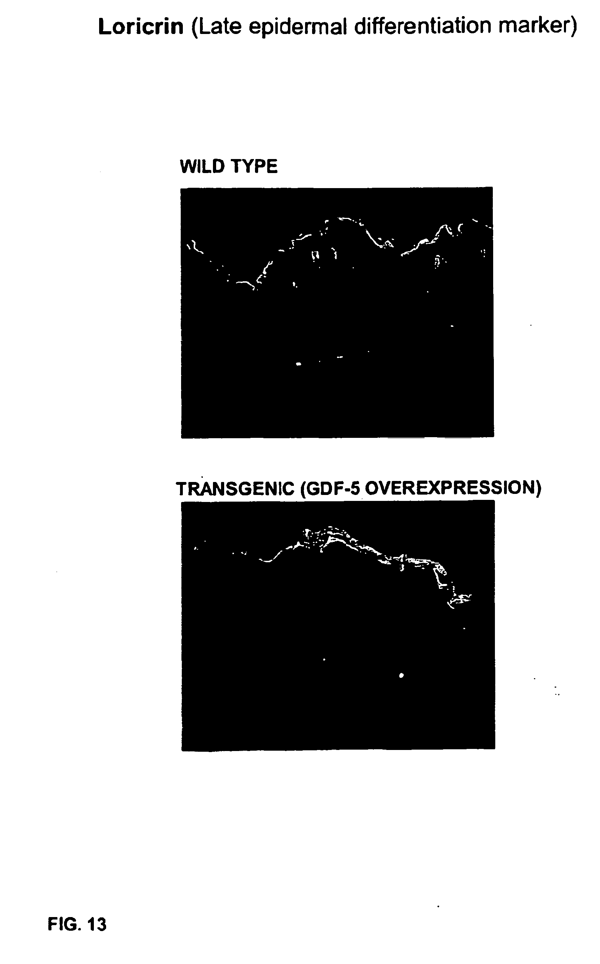

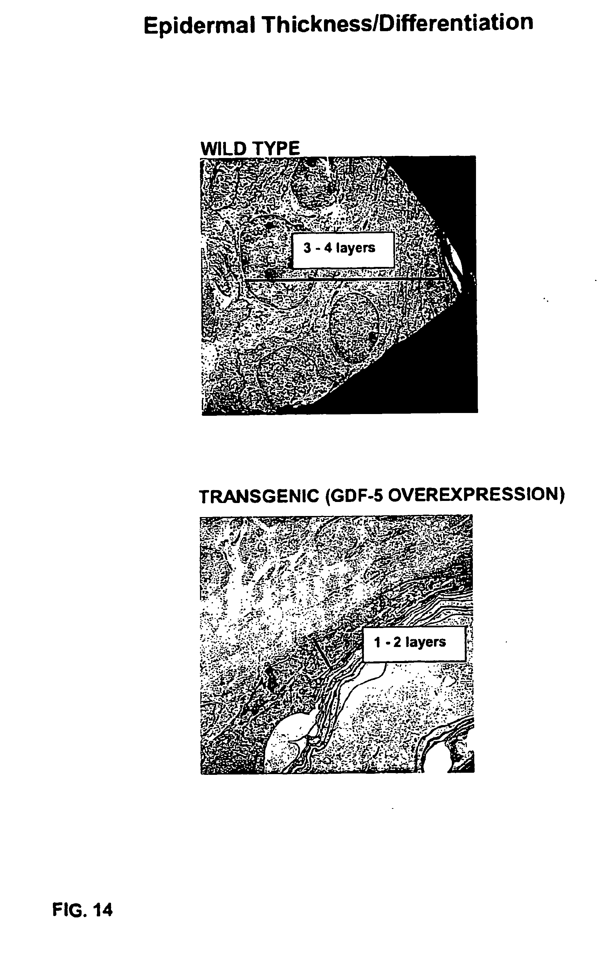

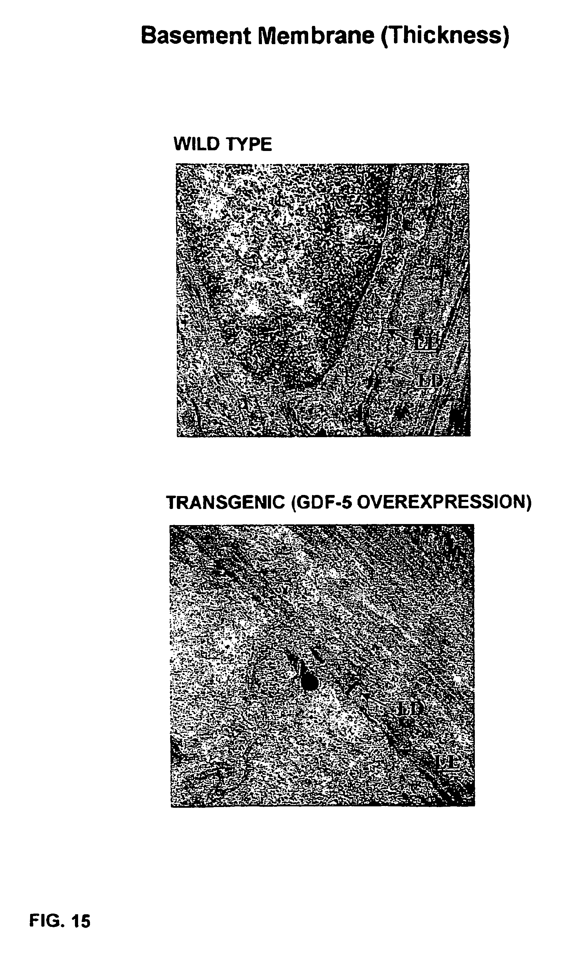

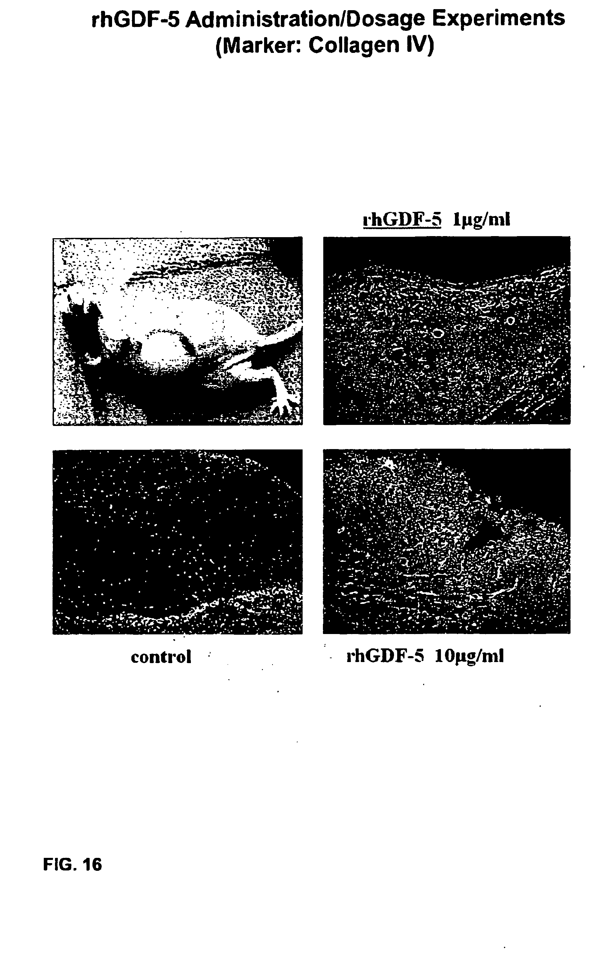

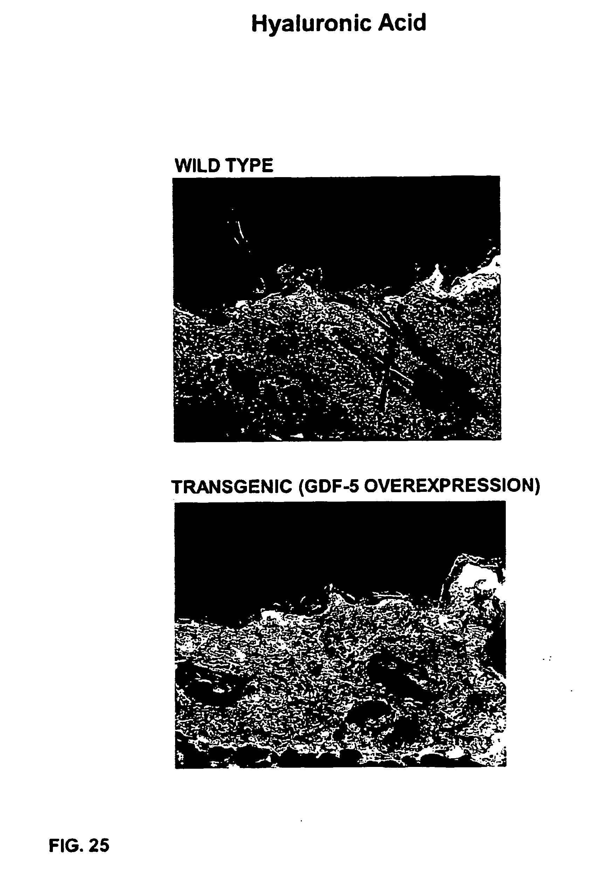

According to the detailed and extensive *in vivo* experiments described in examples 1 and 2 of the present application, artifical increase of the GDF-5 concentration in mammalian skin tissue has significant structural implications on the ECM scaffold. More precisely, i.e. the following important effects can be observed:
- strong increase of prominent extracellular matrix proteins: collagen type I (FIG. 6), collagen type III (FIG. 7), collagen type IV (FIG. 16), elastin (FIG. 8), fibrillin-2 (FIG. 12) and decorin (FIG. 9);
- significant rise in the concentration of basement membrane proteins: collagen type IV (FIG. 10), collagen type VII (FIG. 11) and flbrillin-2 (FIG. 12);
- distinct enhancement of epidermal differentiation marker loricrin (FIG. 13);
- enhanced content of hyaluronic acid (FIG. 25);
- overall reduction of epidermal thickness (FIG. 14)
- considerable strengthening of basement membrane (FIG. 15).

Although the GDF-5-mediated effects also intertwine in a synergistic manner, it is remarkable that each observed alteration individually affects ECM composition and initiates a discrete regeneration action on dermal cells. The subject-matter of the invention is defined in the present claims. In one embodiment of the invention, the disclosed cosmetic, non-therapeutic methods which utilize GDF-5-related proteins can be used for the prevention, deceleration or reversal of dermal aging processes selected from chronological aging and photoaging. The growth factor-mediated increase in structural proteins gives rise to reversal of a multitude of degenerative effects. Especially preferred is the amelioration or prevention of wrinkle, the retardation of dermal aging, the thickening of basement membrane and/or the reduction of epidermal thickness.

For a better understanding of the scientific context and the impact of the invention, some relevant literature which summarizes age- and photodamage-related alterations in dermal cells is shortly described hereinafter.

The aging process of the skin relates to intrinsic/chronologic aging and photoaging. Both types of aging, damage to human skin due to repeated exposure to ultraviolet (UV) radiation from the sun (photoaging) and damage occurring because of the passage of time (chronologic aging), result in a number of changes in the structure, mechanical properties and function of skin, ultimately leading to the emergence of wrinkles and a significant reduction of skin elasticity. Primary among the intracellular alterations caused by chronologic and photoaging are considerably lower levels of collagens, decorin and other components of the skin's connective tissue. Studies have i.e. demonstrated that
- collagen type I is reduced in UV-radiated skin by 58% due to increased elaboration of collagen-degrading matrix metalloproteinases and decline of collagen synthesis, (Fisher et al. 1997: Pathophysiology of premature skin aging induced by ultraviolet light. N Engl J Med 337: 1419-1428). A significant reduction in collagen type I is also typical for chronologic aging, as demonstrated by a comparison of dermal fibroblasts isolated from skin of young (18 to 29 years) versus old (80+ years) individuals (Varani et al. 2006: Am J Pathol. 168, 1861-1868).
- a significant increase of collagen type III has been found to be linked with a marked improvement of skin elasticity and a decrease of wrinkle depth in anti-aging studies (Schmidt et al. 1996: Treatment of skin aging with topical estrogens. Int J Dermatol. 35: 669-674).
- ultraviolet B-irradiated, wrinkle-bearing skin is characterized by an enhancement of type IV collagen degradation activity (Inomata et al. 2003: Possible involvement of gelatinases in basement membrane damage and wrinkle formation in chronically ultraviolet B-exposed hairless mouse. J Invest Dermatol. 120,128-34).
- the basal expression of the type VII collagen gene decreases in an age-dependent manner in fibroblasts (Chen et al. 1994: Type VII collagen gene expression by human skin fibroblasts and keratinocytes in culture: influence of donor age and cytokine responses. J Invest Dermatol. 102, 205-209).
- chronic UVB irradiation induces loss of hyaluronic acid from the dermis, thereby contributing to the quiescent phenotype of dermal fibroblasts (Dai et al. 2007: Chronic ultraviolet B irradiation causes loss of hyaluronic acid from mouse dermis because of down-regulation of hyaluronic acid synthases. Am J Pathol. 171, 1451-1461)
- the hyaluronic acid content of human skin decreases in older individuals (Poulsen et al. 1982: Determination of hyaluronic acid, dermatan sulphate, heparan sulphate and chondroitin 4/6 sulphate in human dermis, and a material of reference. Scand J Clin Lab Invest. 42, 545-549).
- elastin degeneration is a prominent histologic marker of chronologic skin aging (Mestre-Deharo et al. 1991: Histologic signs of cutaneous aging. Rev Fr Gynecol Obstet. 86, 425-432).
- keratinocytes synthesize fibrillin-2 and assemble beaded microfibrils concurrently with expression of basement membrane collagen. These observations suggest that keratinocytes co-ordinate the assembly of these distinct structural elements and have important implications for skin remodelling (Haynes et al. 1997: Keratinocytes express fibrillin and assemble microfibrils: implications for dermal matrix organization. Br J Dermatol. 137,17-23.
- immunostainings revealed that decorin is greatly decreased within a photodamaged dermal area (Bernstein et al. 1995: Differential expression of the versican and decorin genes in photoaged and sun-protected skin. Comparison by immunohistochemical and northern analyses. Lab Invest. 72: 662-669).
- a comparison between the skin of an older and a younger group of volunteers (Sauermann et al. 2002: Age related changes of human skin investigated with histometric measurement by confocal laser scanning microscopy in vivo. Skin Res Technol 8, 52-56) demonstrates that epidermal thickness increases in skin tissues of older individuals, whereas the thickness of the basal layer decreases significantly in this group. Thus, it seems reasonable to develop cosmetic compositions which are able to stop or decelerate these age-related thickness changes. As disclosed herein, an increased concentration of GDF-5-related proteins in human skin tissues is very helpful to generate the desired effects. Transgenic animals with dermal GDF-5 overexpression have a 30% thicker basement membrane than wild type animals. The epidermal thickness is significantly reduced (1-2 layers in comparison with 3-4 layers in the wild type).

The results disclosed in this patent application demonstrate that GDF-5 and/or closely related proteins are indeed effective modulators of ECM composition. The concentrations of substances such as collagen type I, collagen type III, collagen type IV, collagen type VII, fibrillin-2, hyaluronic acid, elastin and decorin can be significantly altered and increased by applying the methods and compositions described herein.

The described structural modifications of the extracellular matrix can be evoked by naturally occurring or artificially designed proteins which are very closely related to human growth/differentiation factor 5 (hGDF-5). The term "GDF-5-related proteins" includes functionally similar proteins belonging to the group of vertebrate GDF-5, GDF-6 and GDF-7 proteins as well as recombinant variants thereof. Due to an extraordinary high degree of amino acid sequence identity (see FIG. 2), this group of proteins exhibits comparable biological properties. Common feature of all GDF-5-related proteins is the occurrence of a bioactive cystine-knot-domain with an amino acid identity of at least 60% to the 102 aa cystine-knot domain of human GDF-5/SEQ ID NO: 1 which is sufficient for the biological function of the protein. As can be seen from FIG. 3, the preferred limiting value of 60% separates members of the GDF-5/-6/-7 group from more distantly related GDFs and BMPs. Especially preferred proteins display amino acid identities of at least 70%, 80% or 90% to the 102 aa cystine-knot domain of human GDF-5.

Non-limiting examples for vertebrate and mammalian GDF-5-related proteins are precursors and mature proteins of human GDF-5 (disclosed as MP52 in WO95/04819 and as human GDF-5 in Hötten et al. 1994, Biochem. Biophys Res. Commun. 204, 646-652), recombinant human (rh) GDF-5/MP52 (WO96/33215), MP52 Arg (WO97/06254); HMW human MP52s (WO97/04095), CDMP-1 (WO96/14335), mouse (Mus musculus) GDF-5 (US 5,801,014), rabbit (Oryctolagus cuniculus) GDF-5 (Sanyal et al. 2000, Mol Biotechnol. 16, 203-210), chicken (Gallus gallus) GDF-5 (NCBI accession no. NP_989669), African clawed frog (Xenopus laevis) GDF-5 (NCBI accession no. AAT99303), monomeric GDF-5 (WO 01/11041 and WO 99/61611), human GDF-6/BMP-13 (US 5,658,882), mouse GDF-6 (NCBI accession no NP_038554), GDF-6/CDMP-2 (WO96/14335), human GDF-7/BMP-12 (US 5,658,882), mouse GDF-7 (NCBI accession no AAP97721), GDF-7/CDMP-3 (WO96/143335). Covered by the invention are also GDF-5-related proteins having additional mutations such as substitutions, additions and deletions, as long as these additional mutations do not completely abolish the biological protein activity. Some preferred variants are mutants of GDF-5-related proteins with improved biological activity. For example, one or more residues which are normally present in the human GDF-5 precursor protein (see FIG. 1) are substituted in these mutants by other amino acids: the arginine at position 438 of the human GDF-5 precursor is replaced by glycine, alanine, valine, leucine, isoleucine, methionine or asparagines; and/or serine 439 is replaced by aspartic acid, glutamic acid, glycine, leucine, or isoleucine; and/or asparagine 445 is replaced by serine or threonine. In another high activity mutant, methionine 453 and/or methionine 456 are replaced by alanine, valine, or isoleucine. Also of special interest are mutants in which leucine 441 is replaced by proline.

The biological activities of GDF-5-related proteins can be easily determined with the help of established test systems. Most useful and preferred is a common in vitro test known as alkaline phosphatase (ALP) assay (Takuwa et al. 1989, Am. J. Physiol. 257, E797-E803), which is also described in example 3. GDF-5-related proteins have been demonstrated to increase alkaline phosphatase activity i.e. in ROB-C26 cells (Yamaguchi et al. 1991, Calcif. Tissue Int. 49, 221-225) as described in W095/04819, in embryonic ATDC5 cells (Riken Gene Bank, ROB 0565), in mouse stromal MCHT-1/26 cells, and in HPDL cells as shown in Nakamura et al. 2003, J. Periodontal Res. 38,597-605.

For the present cosmetic methods and uses, GDF-5-related proteins may be applied in both cosmetic compositions. Most administration procedures require compositions having a nearly physiological pH. Unfortunately, GDF-5 and the closely related proteins GDF-6 and GDF-7 feature an uncommon surface charge distribution pattern which is associated with poor solubility at pH values between pH 4 and pH 9. Although GDF-5-related proteins may be administered by means of aqueous solutions having a pH around or below 4, the most efficient way to overcome the solubility problem is the adhesion to a specific colloidal drug carrier of very small particle size. The interaction between growth factor and the selected microstructured carrier efficiently prevents the undesired coagulation of the protein at slightly acid/basic and even at neutral pH.

In principle, various colloidal carriers known in the art may be utilized for cosmetic compositions. Suitable carriers are extensively described in the literature (see i.e. Barrat et al. 2003: Colloidal drug carriers: achievements and perspectives. Cell. mol. life sci. 60, 21-37). However, because of the anomalous surface charge distribution of GDF-5, testing and selection of a colloidal carrier material which is optimized for the GDF-5/GDF-6/GDF-7 family of growth factors is advisable. Among several others, frequently used colloidal carriers are i.e. liposomes, mixed micelles, microemulsions, lipid microparticles (LMP), and polymeric nanoparticles, which are shortly characterized hereinafter:
Liposomes are very simple structures consisting of one or more lipid bilayers of amphiphilic lipids, i.e. phospholipids or cholesterol. The lipophilic moiety of the bilayers is turned towards each others and creates an inner hydrophobic environment in the membrane. The size of liposomes varies from 20 nm to few µm.

Mixed micelles are detergent structures which are composed of bile salts, phospholipids, tri, di- and monoglycerides, fatty acids, free cholesterol and fat soluble micronutrients. As long-chain phospholipids are known to form bilayers when dispersed in water, the preferred phase of short chain analogues is the spherical micellar phase. The interaction between micelles and hydrophobic/lipophilic drugs leads to the formation of mixed micelles (MM). In the human body, they incorporate hydrophobic compounds with low aqueous solubility.

Microemulsions are clear, stable, isotropic liquid mixtures of oil, water and surfactant, frequently in combination with a cosurfactant. The aqueous phase may contain salt(s) and/or other ingredients, and the "oil" may actually be a complex mixture of different hydrocarbons and olefins. In contrast to ordinary emulsions, microemulsions form upon simple mixing of the components and do not require the high shear conditions generally used in the formation of ordinary emulsions. The two basic types of microemulsions are direct (oil dispersed in water, o/w) and reversed (water dispersed in oil, w/o).

Lipid microparticles may be divided into lipid nano- and micospheres. Microspheres are generally defined as small spherical particles made of any material which are sized from about 0.2 to 100 µm. Smaller spheres below 200 nm are usually called nanospheres. Lipid microspheres are homogeneous oil/water microemulsions similar to commercially available fat emulsions. They prepared by an intensive sonication procedure or high pressure emulsifying methods (grinding methods). Simple lipid microspheres consisting of microscopic oil drops in an aqueous dispersion were first investigated for parenteral nutrition (Shenking A. World Rev. Nutr. Diet. 28, 1-111, 1978). Also systems based on aqueous emulsions of soy oil and an outer lecithin (phosphatidycholine) shell have been developed (Mizushima Y. Drugs Exptl. Res. XI (9), 595-600, 1985). The natural surfactant lecithin lowers the surface tension of the liquid, thus acting as emulsifier (Seki et al, J. Controlled Release 28, 352-353).

Polymeric nanoparticles serve as carriers for a broad variety of ingredients. The active components may be either dissolved in the polymetric matrix or entrapped or adsorbed onto the particle surface. Polymers suitable for the preparation of organic nanoparticles include cellulose derivatives and polyesters such as poly(lactic acid), poly(glycolic acid) and their copolymer.

Creation and testing of these and other colloidal drug carriers is a routine matter which can be done without undue burden. However, the inventors have exemplarily created and checked several colloidal drug carriers in combination with GDF-5 (see examples 4 and 5).. According to the tests, polymeric nanoparticles (i.e. poly-(DL)-lactide PLA resomer® R202H, Boehringer Ingelheim, Ingelheim, Germany) are capable of incorporating medium scales of GDF-5-related proteins (up to 66 g/mg). Since cosmetics usually contain lower amounts of active ingredients than pharmaceuticals, nanoparticles of such kind were considered to be appropriate carriers for cosmetic compositions. Also oil-water microemulsions comprising GDF-5 related proteins, glycerol and emulsifying agents have been created and successfully tested (see example 4). Furthermore, some lipid microparticle formulations (LMPs) were specifically designed. According to the test results, LMPs might carry up high amounts (up to 2.5 mg/ml) of GDF-5 at physiological pH (pH 7). Thus, lipid microparticles are suitable carriers of GDF-5-related proteins.

There are numerous natural and synthethic lipids and oils available which might be used as lipid carriers in colloidal formulations such as a LMP formulation. Synthetic lipids and oils are recommendable due to their purity and their known chemical properties. All kinds of synthetic oils/lipids can be used as long as they are biocompatible, for example synthetic oils or saturated esters such as ethyl palmitate, isopropyl palmitate, alkyl myristates such as those of isopropyl, butyl and cetyl, hexyl stearate, triglycerides (i.e. of octanoic or decanoic acids, medium chained tryglycerides such as Miglyol® 812), cetyl ricinoleate, stearyl octanoate (purcelllin oil) and hydrogenated polyisobutene. Due to their well-known biocompatibility and other characteristics, especially common plant oils such as e.g. cottonseed, soybean, sesame, sunflower, safflower, olive, avocado, peanut, walnut, almond and hazelnut oil are appropriate in most cases. Most preferred oils are olive oil, soybean oil and safflower oil which have been successfully tested. Other natural oils are likewise applicable.

In addition, the formulation might comprise an emulsifying agent. Preferred are phospholipids such as e.g. phosphatidylserine, phosphatidylcholine or phosphatidylethanolamine, preferably in a similar total amount (+/- 25 percent w/w, w/v or v/v) as the lipid carrier. A successfully tested commercially available emulsifier is Phospolipon ®, a soy phospholipid fraction comprising at least 80% phosphatidylcholine. Other emulsifiers are also possible, e.g. distilled monoglycerides, mono- & diglycerides, acetic acid esters of monoglycerides, organic esters of monoglycerides, sorbitan esters of fatty acids, propylene glycol esters of fatty acids and polyglycerol esters of fatty acids.

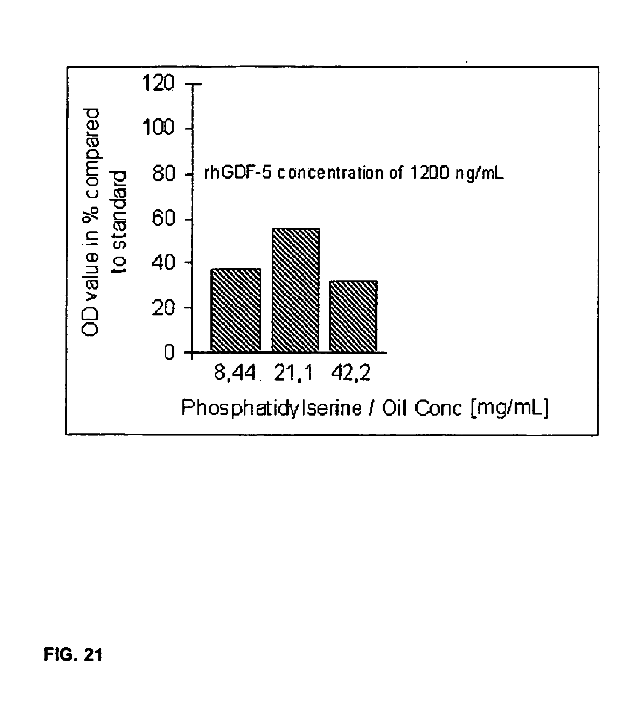

The optimal concentration of oil and/or emulsifying agent contributes to the effective generation of bioactive GDF-5-containing lipid microparticles. Exemplarily, groups of GDF-5 LMP formulations containing low (5-15 mg/ml), intermediate (16-30 mg/ml) and high (31-50 mg/ml as well as up to 100 mg/ml) concentrations of oil and identical amounts of emulsifier phosphatidylserine in succinate buffer were tested in the ALP assay system. All formulations proved to be bioactive. However, the activity peaked in the intermediate group (see FIG. 21). According to these results, the LMP formulations may comprise concentrations of oil and/or emulsifying agents in the range between 5 and 100 mg/ml. The preferred LMP formulations for use according to the invention comprise concentrations of oil and/or emulsifying agents between 16 and 30 mg/ml. Most preferred are concentrations between 20 and 25 mg/ml.

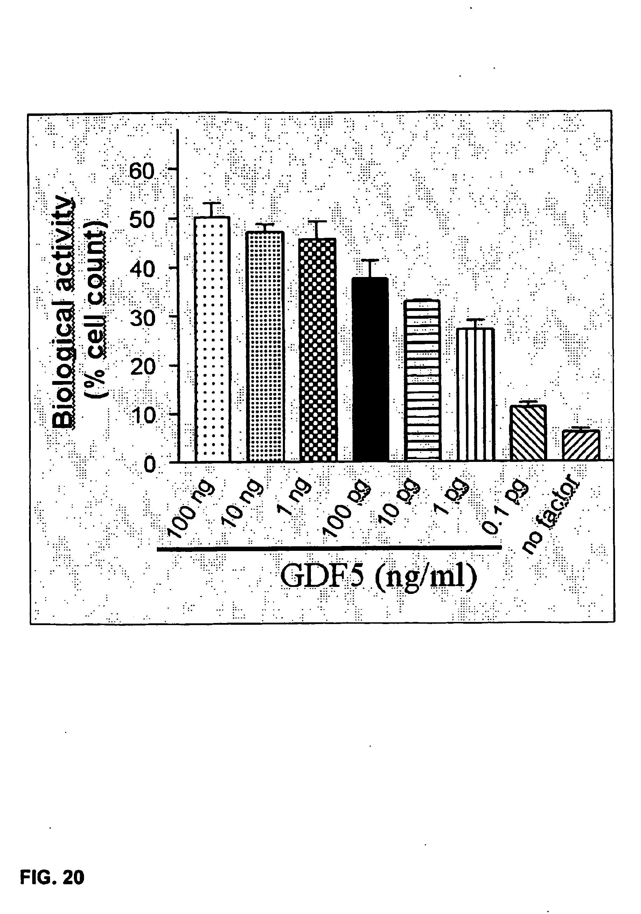

The concentrations of GDF-5-related proteins in the compositions for use according to the invention should be chosen in dependency on the mode and period of application. Basically, GDF-5-related proteins are highly potent cytokines which are capable of eliciting effects even in exiguous quantities. As easily determinable with the help of different biological assay systems such as i.e. the alkaline phosphatase assay described herein, a concentration of 0.1 pg GDF-5 per ml of the respective solution is sufficient to cause biological actions (see dose determination according to FIG. 20). Accordingly, low concentrations, i.e. ranging from 0.1 pg/ml to 1 ng/ml or less, are preferred if the cosmetic compositions of the invention are repeatedly administered, i.e. in terms of daily applied skin or beauty cremes. However, maximum effects are achievable with higher growth factor concentrations. In the assay system shown in FIG. 20, 1 - 100 ng/ml GDF-5 are most effective. An independent dose response analysis of GDF-5 action utilizing a wide range of serial dilutions (0.3 - 80 ng/ml, Farkas et al. 1997, Neurosci. Lett. 236, 120-122) gave optimal results at a concentration of 20 ng GDF-5 per ml. *In vivo* skin models (see i.e. example 2/Fig. 16), commonly use high doses of 1 - 10 µg/ml. Therefore, in a preferred embodiment of the invention, the cosmetic compositions contain GDF-5 related proteins in concentrations of between 0.1 pg/ml and 10 µg/ml. Preferred total doses of GDF-5 related proteins in case of one time administrations (e.g. injections) range from 10 ng to 10 µg.

Because of their small size and good biocompatibility, the compositions can be generally administered by a variety of routes which are well known in the art. Cosmetic compositions may be preferably applied topically, subcutaneously or transdermally.

The compositions might also comprise other bioactive protein(s) in addition to GDF-5-related proteins. It has been shown that TGF-ß increases the size of regenerated dermis and stabilizes the dermoepithelial junction (Fitzpatrick and Rosen, J. Cosmet Laser Ther, 5: 25-34 (2003)). A cocktail (TNS Recovery Complex, SkinMedica, Inc. Carlsbad, CA, USA) containing seven cytokines (VEGF, IL-6 and -8, HGF, PDGF-a, GCSF, and TGF-ß1) derived from neonatal foreskin fibroblasts was tested in a multicenter study. Evaluation showed improvement in skin texture, and decreased wrinkling (Rokhsar, C.K. et al., Dermatol. Surg. 31: 1166-1178 (2005)). Recombinant epidermal growth factor (ReVive Skincare); and N-furfuryladenine (kinetin) plant growth factor are also on the market. All these proteins may be used together with the GDF-5-related proteins, Other proteins which act synergistically if combined with GDF-5-related proteins are disclosed in the literature/patents, I.e. in WO 99/15191. Preferred are neurotrophins, hedgehog proteins and proteins of the transforming growth factor family, including but not limited to TGF-alpha's, TGF-beta's, activins, BMP's and GDF's. Especially preferred is a combination with any one of EGF, TGF-ß1, TGF-ß2, TGF-ß3, NGF and/or GDNF.

Other acceptable components in the compositions are:
- Retinoids (vitamin A derivatives) which preserve the integrity of mucosal/ epithelial surfaces;
- Hydroxy acids (organic carboxylic acids further classified into alpha hydroxy acids (AHA) and beta hydroxyl acid (BHA)) which enhance epidermal shedding, i.e. glycolic acid, lactic add, citric acid, mandelic acid, malic add, and tartaric acid;
- Antioxidants which counteract the harmful effects of free radicals, i.e. vitamin C, vitamin E, panthenol, lipoic acid, ubiquinone, niacinamide, dimethylaminoethanol, spin traps, melatonin, catalase, glutathione, superoxide dismutase, peroxidase, glucpyranosides, polyphenols, cysteine, allantoin, furfuryladenine, uric acid, and carnosine;
- Depigmenting agents which alleviate hyperpigmentation, i.e. N-acetyl-4-S-cysteanimylphenol, kojic acid, arbutin, azaleic acid, paper-mulberry compound, chemical peeling agents (resorcinol, salicylic acid), Kligman's formula, Pathak's formula, and Westerhofs formula;
- Botanicals, i.e. chamomile, ginseng, Gingko biloba, curcumin, glycyrrhizin, capsaicin, and aloe vera;
- Glycosaminoglycans which support epidermal regeneration, i.e. hyaluronic acid;
- Anticellulites which mediate lipolysis, i.e. beta-adrenergic stimulators such as theobromine, theophylline, aminophylline, caffeine, epinephrine and alpha1-adrenergic stimulators such as yohimbine, piperoxane, and phentolamine;
- Enzymes such as papaine and DNA repair enzymes;
- Hormones, i.e. estrogens, progesterone, testosterone, and growth hormone;
- Antimicrobial agents, i.e. triclosan, chlorhexidine, povidone iodine, hydrogen peroxide, antidandruff preparations, zinc pyrithione;
- Chemical UV filters, i.e. 3-benzylidene camphor (3-BC) or 4-methylbenzylidene camphor (4-MBC);
- Furthermore buffers, stabilizers, preservatives, reducing agents, antioxidant chelating agents, agents that modify isotonicity, deodorants, anaesthetics, adjuvants and solubility-enhancing additives.

These are only non-limiting examples of possible additives, and a worker skilled in the art may easily add other excipients which are in use in cosmetic preparations or which are generally regarded as safe.

It is further preferred that cosmetic compositions comprising GDF-5-related proteins are administered in typical forms for dermal products. Established and standardized formulations which are well known in the art include i.e. lipid microparticle formulations, water-in oil and oil-in-water microemulsions, lotions, gels, creams, ointments, aerosol sprays, lipsticks, plasters, facial packs, bathing agents, facial cleansing agents, bath detergents, bathing soaps, sun creams, sun lotions, self tanning lotions, after sun lotions, oil-in-water emulsions, water-in-oil emulsions, w/o/w emulsions, o/w/o emulsions and milky lotions.

The compositions can be prepared under N₂ (g) using buffers bubbled with N₂ (g) to remove dissolved oxygen or to use a vacuum to remove dissolved gases and protect the lipid components and the GDF-5-related protein from oxidation. Alternatively, other inert gases such as argon might be used.

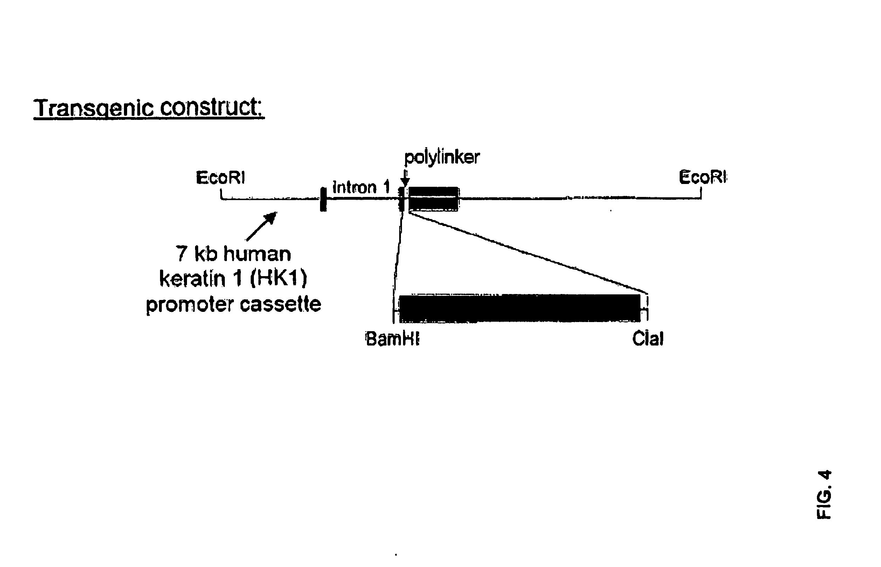

As demonstrated in example 1 together with FIG. 4 - 15, another effective method to carry out the invention is to use a transgenic approach. Since gene therapy strategies require the transfer of genetic material into target cells, a further subject matter of the present invention is the use of a nucleic acid encoding a GDF-5-related protein within the context of the present invention. The nucleic acid can be a DNA sequence and/or a RNA sequence, as long as a GDF-5-related protein as defined herein is obtainable from this nucleic acid upon expression. Base triplets coding for amino acids and the degeneracy of the genetic code are generally known. In the present example 1, a transgenic experiment was performed in accordance with a strategy originally published by Greenhalgh et al. (Oncogene 8 (1993), 2145-2157) and Sellheyer et al. (Proc. Natl. Acad. Sci. USA 90 (1993), 5237-5241). In the described example, a skin-specific promoter (human keratin 1 promoter) is used. Although the use of skin-specific promoters is preferred, other promoters are likewise applicable. As a matter of course, also other gene transfer strategies and vectors which are known in the art can be utilized. Many of these gene transfer vectors are based on modified RNA and DNA viruses (derived from retroviruses, adenovirus, adeno-associated virus, herpesvirus, poxvirus and others) and have been selected as gene delivery vehicles because of their ability to efficiently deliver foreign genes associated with efficient gene expression. Viral vectors are employed in more than 70% of clinical gene therapy trials worldwide. Current and emerging virus-based genetic engineering strategies for the delivery of therapeutic molecules are discussed in Mancheno-Corvo et al. 2006: Viral gene therapy. Clin Transl Oncol. 8, 858-67. However, also non-viral vectors and transfer techniques can be used, i.e. cationic carriers (see Pietersz et al. 2006: Structure and design of polycationic carriers for gene delivery. Mini Rev Med Chem. 6, 1285-1298), electroporation or liposomes (Miyazaki et al. 2006: Gene transfer using nonviral delivery systems. Perit Dial Int. 26, 633-640).

The following non-limiting examples together with the figures and sequence protocols are intended to further illustrate the invention.

SEQ ID NO:1 shows the protein sequence of the human GDF-5 precursor.

SEQ ID NO:2 shows the DNA sequence of the human GDF-5 precursor.

SEQ ID NO:3 shows the 120 aa protein sequence of mature human GDF-5. If recombinantly produced, the protein may alternatively consist of 119 aa, thus beginning with the second aa (proline) of SEQ ID NO:3.

SEQ ID NO:4 shows the 120 aa protein sequence of mature human monomeric GDF-5. The protein may alternatively consist of 119 aa, thus beginning with the second aa (proline) of SEQ ID NO:4.

### FIGURES:

FIG. 1 shows additional features of the human GDF-5 precursor protein according to SEQ ID NO:1:
   aa 001-381 pre-prodomain (bold letters)
   aa 001-027signal peptide (bold and underlined)
   aa 382-501 mature protein part
   aa 400-501 cystine-knot-domain (underlined)
FIG. 2 shows a comparison of the 102 aa cystine-knot domains of human GDF-5 (SEQ ID NO:1), human GDF-6 (sequence 2 from patent US 5,658,882) and human GDF-7 (sequence 26 from patent US 5,658,882). Amino acid residues which are identical in all three molecules are highlighted in black.
FIG. 3 shows a table with the sequence identities of cystine-knot domains of several known BMPs and GDFs to the cysteine-knot-domain of human GDF-5.
FIG. 4 is a schematic diagram of the basic genetic construct of example 1 which consists of a 7 kb human keratin 1 promoter cassette containing a human GDF-5 (hMP52) open reading frame.

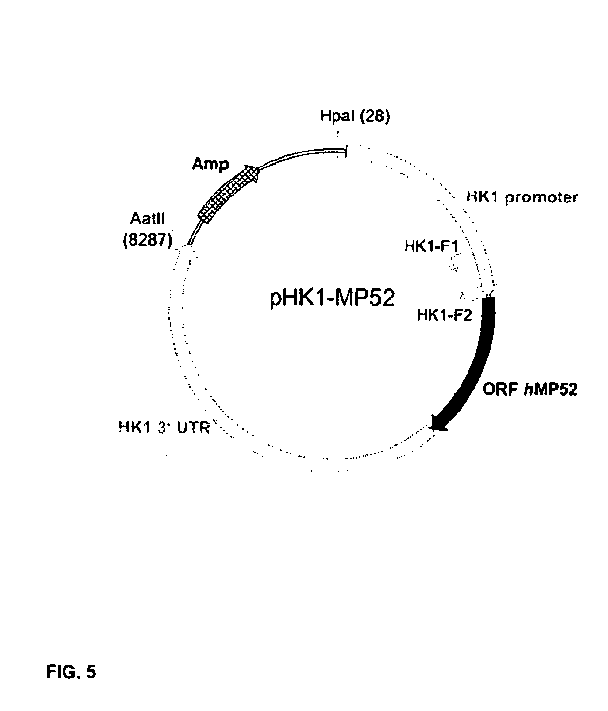

FIG. 5 is a map of the transgenic vector pHK1-MP52 according to example 1.
FIG. 6 shows representative immune fluorescence images visualizing the amount and distribution of collagen type I in the ECM of wild type and GDF-5 overexpressing mice according to example 1. Samples were taken from the back skin of 6 months old mice. Collagen I is more densely packed in the extracellular matrix of transgenic animals. A semi-quantitative evaluation of the staining intensity supports a significant (approx. 10 - 20%) overall increase of collagen I deposition.
FIG. 7 shows representative immune fluorescence images visualizing the amount and distribution of collagen type III in the ECM of wild type and GDF-5 overexpressing mice according to example 1. Samples were taken from the back skin of 5 months old mice. Collagen III is more densely packed in the extracellular matrix of transgenic animals. A semi-quantitative evaluation of the staining intensity supports a significant (approx. 8 - 15%) overall increase of collagen I deposition.
FIG. 8 shows representative immune fluorescence images visualizing the amount and distribution of elastin in the ECM according to example 1. Samples were taken from the back skin of 11 months old mice. Significantly more elastin is deposited in skin tissues of transgenic animals.
FIG. 9 shows representative immune fluorescence images visualizing the amount and distribution of decorin in the ECM of wild type and GDF-5 overexpressing mice according to example 1. Decorin is a collagen-associated structural component of the extracellular matrix. Samples were taken from the belly skin of 11 months old mice. Evaluation of the staining intensity indicates that a considerable higher amount of decorin is present in the skin of transgenic animals.

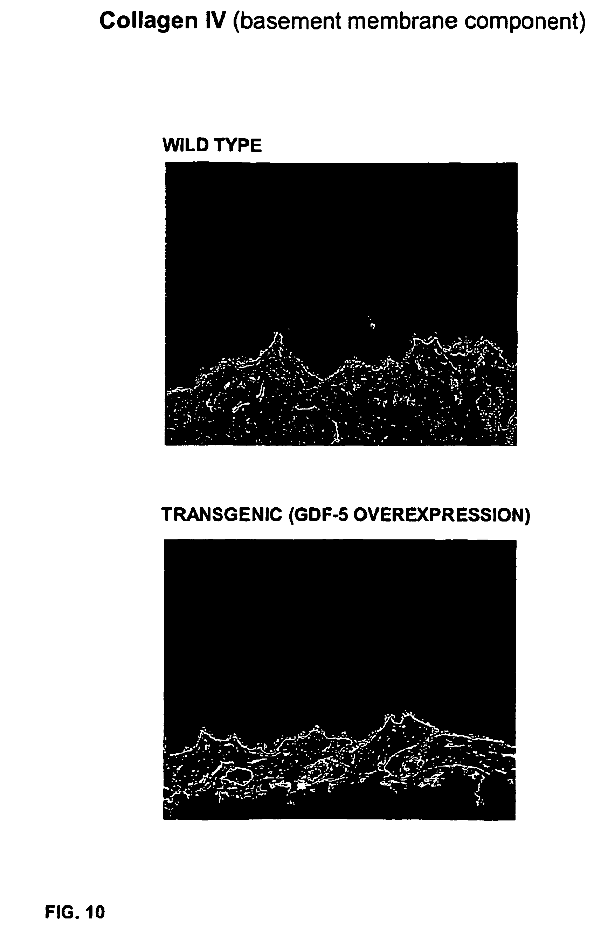

FIG. 10 shows representative immune fluorescence images visualizing the amount of collagen type IV in the basement membranes of wild type and GDF-5 overexpressing mice according to example 1. Samples were taken from the back skin of 11 months old mice. Results demonstrate a remarkable thickening of the basement membranes in skin samples of transgenic animals. Deposition of collagen IV is clearly enhanced in basement membranes of transgenic mice.

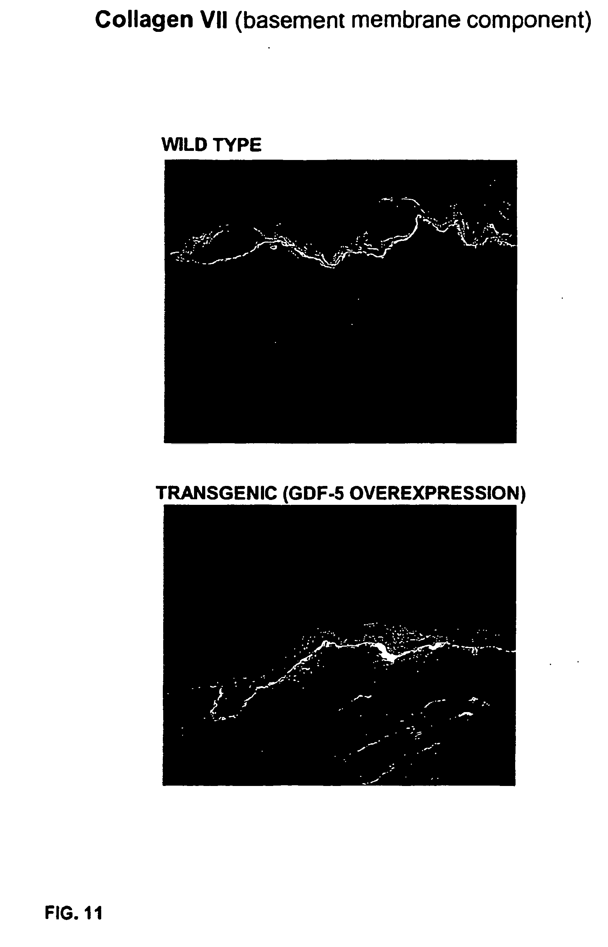

FIG. 11 shows representative immune fluorescence images visualizing collagen type VII in the basement membranes of wild type and GDF-5 overexpressing mice according to example 1. Samples were taken from the back skin of 11 months old mice. The skin of transgenic animals comprises thicker basement membranes with elevated concentrations of collagen VII.
FIG. 12 shows representative immune fluorescence images visualizing the amount and distribution of fibrillin-2 in skin samples of wild type and GDF-5 overexpressing mice according to example 1. Samples were taken from the back skin of 11 months old mice. Fibrillin-2 i predominantly located in basement membranes and also an ECM component. Membranes of transgenic animals are characterized by significantly higher concentrations of fibrillin-2, especially in the basement membranes.
FIG. 13 shows representative immune fluorescence images visualizing loricrin in skin samples of wild type and GDF-5 overexpressing mice according to example 1. Samples were taken from the back skin of 11 months old mice. Loricrin is an important marker of late epidermal differentiation. As indicated by a stronger fluorescence signal in the epidermis of transgenic animals, GDF-5 clearly acts as a differentiation factor in dermal tissues with profound effects on structural proteins and other cell components.
FIG. 14 shows representative electron microscopy images documenting the GDF-5-induced epidermal thickness changes in transgenic mice according to example 1. Samples were taken from the back skin of 11 months old mice. Whereas three to four epidermal cell layers are present in wild type animals, dermal samples of GDF-5 overexpressing mice comprise 1-2 epidermal layers only.
FIG. 15 displays representative electron microscopy images showing the GDF-5-induced thickening of basement membranes in skin samples of transgenic animals according to example 1. Samples were taken from the back skin of 11 months old mice. The results indicate that basement membranes of GDF-5 overexpressing mice (with lamina lucida [LL] and lamina densa [Ld]) are approx. 30% thicker than in wild type animals.
FIG. 16 shows representative immune fluorescence images documenting the dose-dependant GDF-5-induced enhancement of collagen type IV in mice skin according to example 2. The upper left picture shows a mouse with a transplanted GDF-5 containing gel carrier (covered with a silicon hat) according to example 2. Significant dose-dependant epithelial alterations were observed three weeks after adminstration of 1 or 10 µg GDF-5. Neovascularization as well as enhanced formation of various structural cell components (i.e. picture: collagen IV) was visualized via standard immunfluorescence techniques.

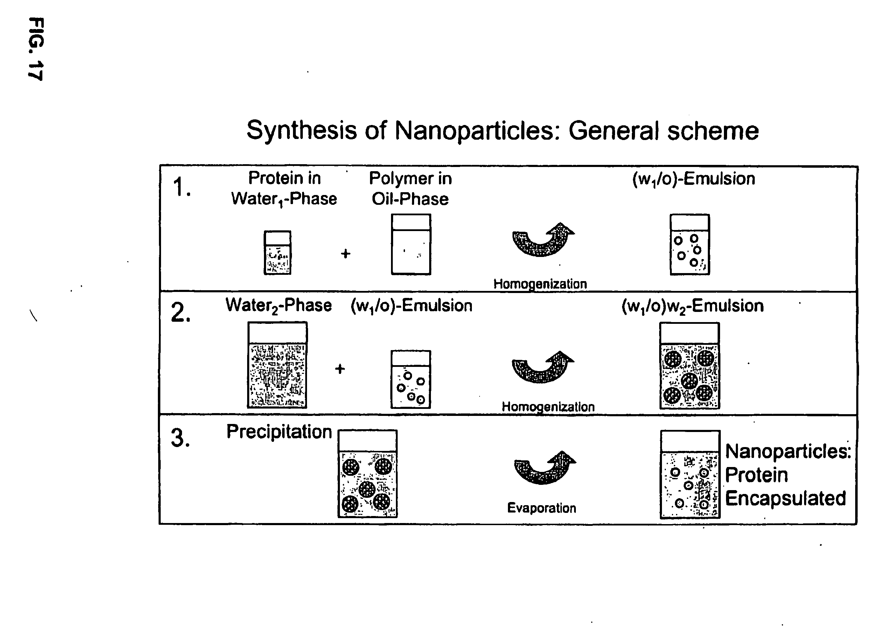

FIG. 17 shows the general synthesis scheme of polymeric nanoparticles (double-emulsion method) as described in example 4.

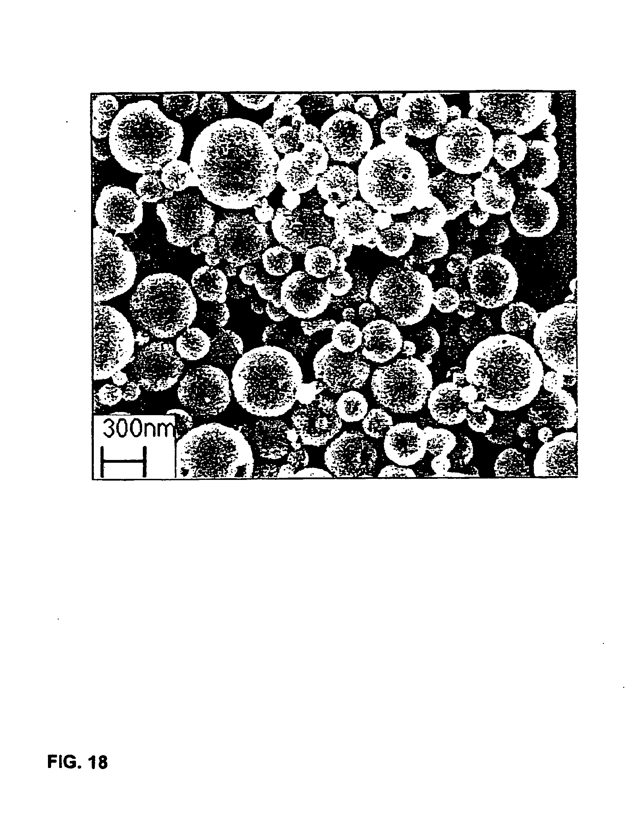

FIG. 18 shows a reflection electron microscopy (REM) image of Resomer® R202H polymeric nanoparticles according to example 4.

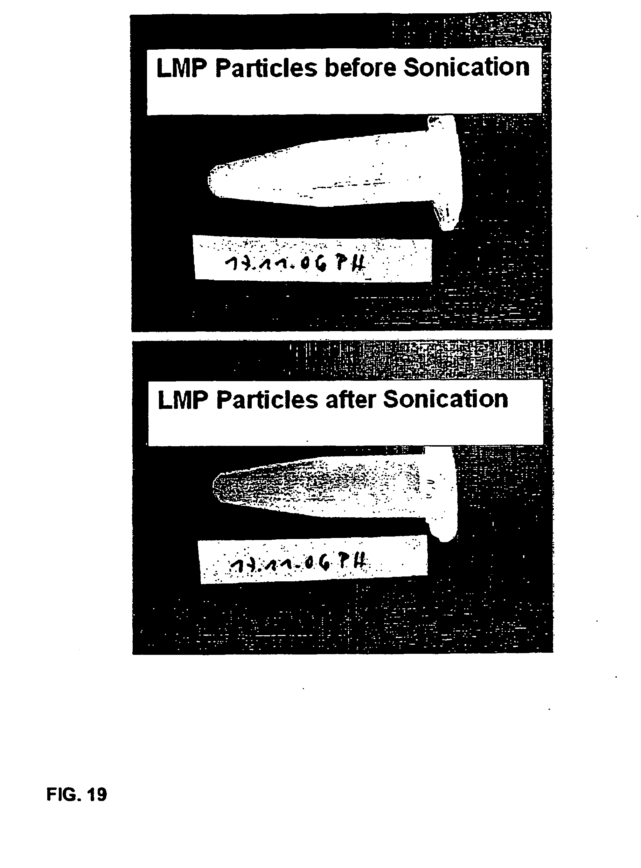

FIG. 19 demonstrates the effect of sonication which is mandatory in the production of lipid microparticle (LMP) compositions according to example 4. Samples (upper picture) containing 22.93 µl olive oil (21.1 mg), an equal weight of phosphatidylserine, and 956 µl of 50 mM succinate buffer (pH 4.25), were sonicated for 1 hour. After sonication, the previously milky emulsion (upper picture) became transparent with an amber colour when held up to the light (lower picture), indicating that the emulsion spheres were getting smaller and reached the expected diameter of approximately 50 nm.
FIG. 20 shows results of a GDF-5 *in vitro* dose determination study (promotion of cellular survival). GDF-5 is effective over the whole range of tested concentrations (0.1 pg/ml to 100 ng/ml).
FIG. 21 shows a comparison of the biological activities of three groups of GDF-5 lipid microparticle formulations comprising different concentrations of phosphatidylserine and olive oil (low/group 1: 5-15 mg/ml, represented in the figure 3 by 8.44 mg/ml; intermediate/ group 2: 16-30 mg/ml, represented by 21.1 mg/ml, high/group 3: 31-50 mg/ml; represented by 42.2 mg/ml). The groups were tested in the ALP assay system according to example 3. OD= optical density.

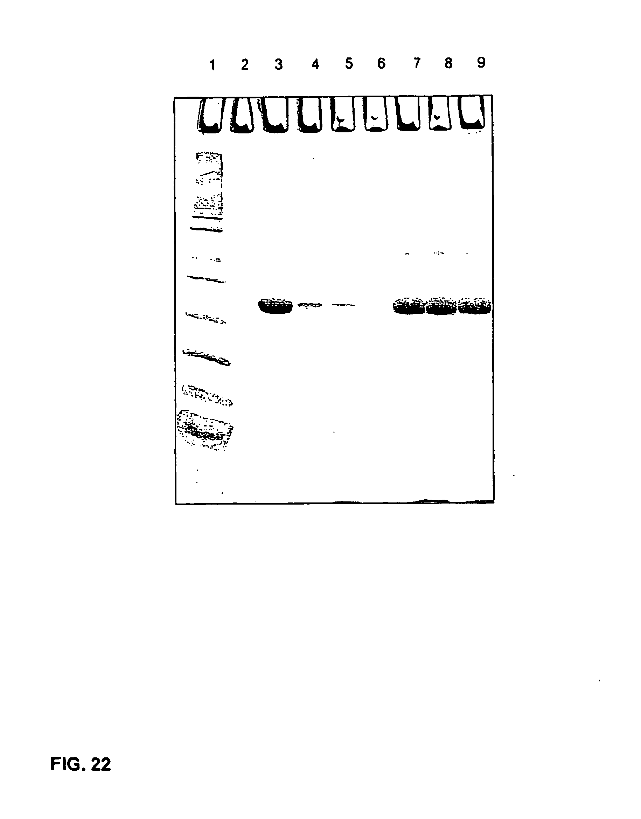

FIG. 22 shows the results of protein size separation via SDS-Page according to example 5. LMP pellets with encapsulated GDF-5 were heated for 10 min at 80° C to solubilize the lipids. Only very few degradation products are visible, thus demonstrating stability of the GDF-5 protein. Lanes from left to right: 1:marker protein, 3-5: GDF-5 reference standard 5 µg, 0,25 µg, 0,025 µg, 7-9: GDF-5 LMP samples (5 µg each).

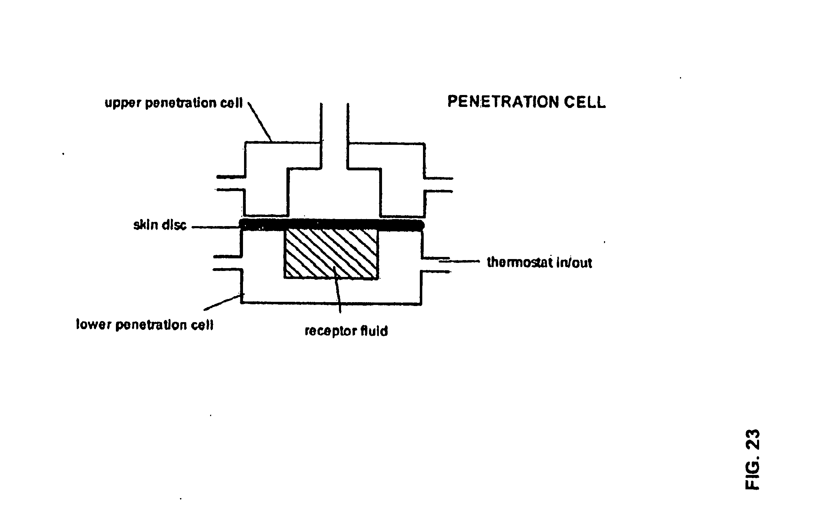

FIG. 23 shows a general scheme of a penetration cell for the assessment of dermal absorption and percutaneous penetration of cosmetic compositions according to example 5.

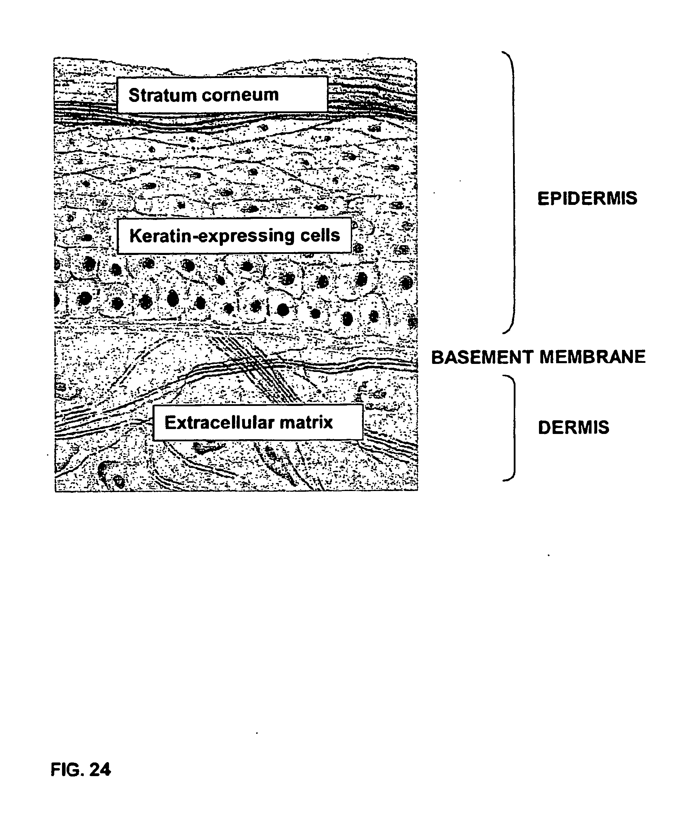

FIG. 24 demonstrates the general structure and organization of human skin.
FIG. 25 shows representative immune fluorescence images documenting the elevated content of hyaluronic acid (red stain, i.e. arrow) as a water binding substance in the extracellular matrix of GDF-5 overexpressing animals according to example 1. Samples were taken from the back skin of 11 months old mice. In comparison with wild type mice, quantitative evaluation of the staining intensity indicates a significantly (approx. 20 - 30%) higher concentration of hyaluronic acid in the skin of transgenic mice, thus considerably increasing the moisture content of the skin.

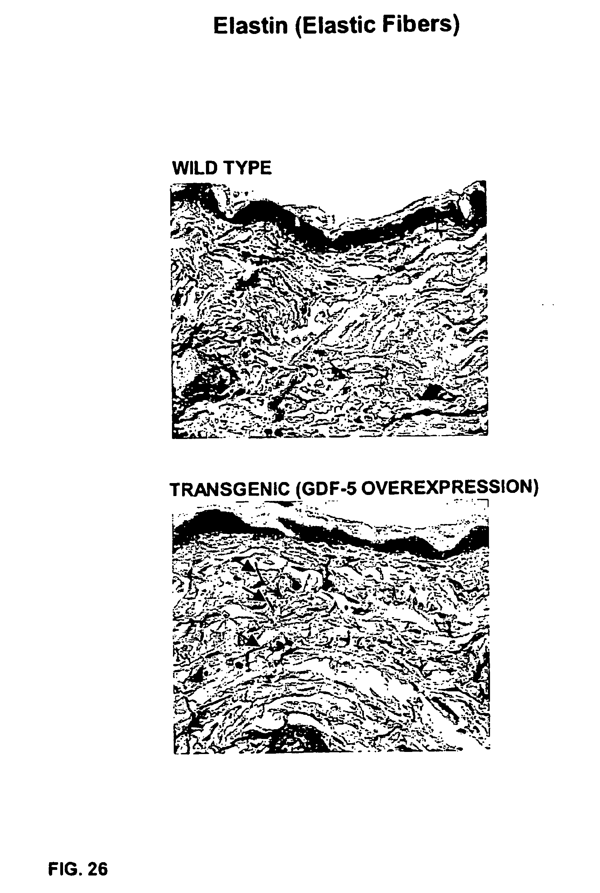

FIG. 26 shows a van Gieson elastin staining of skin samples of wild type and GDF-5 overexpressing transgenic animals according to example 1 (see e.g. Garvey, Winsome et al (1991): A modified Verhoff Elastic Van Geison Stain. J. Histotech. 14, (2), 113-114). Samples were taken from the back skin of 11 months old mice. High numbers of elastic fibers are predominantly present in the transgenic samples, which appear as bluish-black rods (see arrows).

### EXAMPLES:

### Example 1: GDF-5-mediated alterations of skin structure in vivo

This set of experiments, utilizing a transgenic *in vivo* mouse model, was designed to determine whether an increased intracellular concentration of GDF-5-related proteins is useful to enhance the content of crucial structural ECM components such as i.e. collagens, fibrillins, elastin and decorin. The concentrations of these scaffold substances decline during life, i.e. as a result of aging, photodamage or various disorders.

Following the strategy used by Greenhalgh et al. (Oncogene 8 (1993), 2145-2157) and Sellheyer et al. (Proc. Natl. Acad. Sci. USA. 90 (1993), 5237-5241), genetic constructs for the overexpression of human GDF-5 (MP52) in the proliferative basal compartment of the epidermis were created. The basic construct (FIG. 4) consists of a human GDF-5 (hMP52) open reading frame (ORF) which was directionally placed inside a 7 kb human keratin 1 (HK1) promoter cassette by using artificially created BamHI and Clal restriction sites. Subsequently, an EcoRI fragment of the HKI promoter comprising the GDF-5 (hMP52) ORF was subcloned into a plasmid vector and resulted in transgenic vector pHK1-MP52 (FIG. 5).

A transgenic HK1-GDF-5 construct was amplified and isolated from the plasmid pHK1-MP52 via Hpal and AatII digestion. The 8.2 kb fragment was then purified, filtered using a micro-spin column (Ultrafree-MC, Millipore) and diluted to a concentration of 5 ng/µl in microinjection buffer. 3 week old females mice (strain CB6F1/Charles River Laboratories) were superovulated and mated with male C57BL6 mice (Charles River Laboratories). The resulting fertilized CB6F2 oocytes were collected from the oviduct of plugged female mice and cultured until two clear pronuclei were visible. The purified transgenic construct was microinjected into the male pronucleus and injected embryos were cultivated overnight to the two-cell stage. 263 two-cell embryos were then implanted into the oviduct of pseudopregnant foster mothers at 0.5 days post-coitum. In total, three transgenic founder animals were subsequently identified. Results of the DNA microinjection revealed that 3/40 (7.5%) animals had integrated the transgenic DNA, a figure which is compatible with the statistical 5-25% success rate of this technology. Each of the transgenic founder animals were crossed with wild type mice testing them for germ line transmission. Among the F1-generation, transgenic mice could be identified by PCR. Transgenic mice show reduced hair coat and significant changes in skin structure but no gross behavioural abnormalities (FIG. 6 - 15). Cryosections from back or belly skin of 5 and 11 month old F1 animals were examined by standard immmunofluorescence and electron microscopy techniques.

Skin cryosections revealed a strong increase of prominent extracellular matrix components such as collagen type I (FIG. 6), collagen type III (FIG. 7), elastin (FIG. 8 and FIG. 26), fibrillin-2 (FIG. 12) and decorin (FIG. 9), a distinct rise in the concentration of basement membrane proteins collagen type IV (FIG. 10), Collagen type VII (FIG. 11) and fibrillin 2 (FIG. 12), an enhancement of epidermal differentiation marker loricrin (FIG. 13), an overall reduction of epidermal thickness (FIG. 14), a considerable strengthening of the basement membrane (FIG. 15) and an enhanced content of hyaluronic acid (FIG. 25).

### Example 2: In vivo Matrix inserted surface transplantation assay

With the objective to study the effects of directly administered GDF-5 protein on dermal tissues, a matrix inserted surface transplantation assay was used. Briefly, gel carriers were loaded with different amounts (10 animals each with 1 µg/ml or 10 µg/ml) of recombinant human GDF-5. A circular area of skin was excised from the back of nude mice and covered with vaseline gauze. Subsequently, gel carriers containing GDF-5 were mounted between 2 rings, covered with a silicon hat and transplanted on back muscle fascia (FIG. 16). 1 to 4 weeks after transplantation, dermal samples were taken and examined via cytohistochemistry.

Significant dose-dependant epithelial alterations were observed 3 weeks after application of 1 or 10µg GDF-5 (FIG.16). Neovascularization as well as enhanced formation of structural cell components (collagen I, IV, VII, fibrillin, elastin, decorin, hyaluronic acid) was visualized via standard immunfluorescence techniques. In summary, direct administration of GDF-5 to skin tissues evoked comparable effects as the overexpression of GDF-5 in transgenic skin of mice (example 1).

### Example 3: Alkaline phosphatase (ALP) Testing of biological activity

The biological activitiy of GDF-5-related proteins and colloidal formulations thereof can be easily determined with the help of established test systems. Most useful and preferred is the common alkaline phosphatase (ALP) assay (Takuwa et al. 1989, Am. J. Physiol. 257, E797-E803). In this in vitro test system, the biological activity of GDF-5 related growth factors is measured after co-culture of different protein concentrations with osteogenic/chondrogenic cells. GDF-5 and related proteins with osteo/chondrogenic potential increase the alkaline phosphatase (ALP) expression in these cells, e.g. ATDC-5, ROB-C26 or MCHT-1/26 cells. The ALP activity in these cell lysates is determined by a colorimetric assay. The reaction is based on the hydrolysis of p-Nitrophenylphosphate (PNPP) to p-Nitrophenole, which becomes visible under alkaline conditions as the yellow p-Nitrophenolanion. The aim was to measure the activity of the tested LMP formulations by comparison of the ALP activity obtained with known concentrations of GDF-5 reference standard.

In a standardized ALP assay, 1x10⁴ cells of ATDC-5 of MCHT1/26 cells were incubated overnight in 96-well plates in cell culture medium (alpha-MEM, Penicilline/Streptomycine, 2 mM L-glutamine, 10% FCS) at 37°C, 5% CO₂, H₂O-saturated. The next day, cells were stimulated with the GDF-5-related proteins or formulations thereof for 72 hrs with indicated ligand concentrations. The cells were subsequently washed with PBS (phosphate buffered saline). Cell lysis was performed in 100 µl alkaline lysis buffer 1 (0,1M glycine, pH 9.6, 1% NP-40, 1 mM MgCl₂, 1mM ZnCl₂) for 1 h at room temperature. Then 100 µl alkaline lysisbuffer 2 was added (0.1 M glycine, pH 9.6, 1mM MgCl₂, 1mM ZnCl₂ + 2mg/ml PNPP). The plates were incubated at 37°C, 5% CO₂, H₂O-saturated. The ALP-reaction was stopped afterwards with 100 µl of 30g/l NaOH and finally the optical density was measured with an automatic microplate reader at 405 nm under consideration of blank value subtraction.

### Example 4: Development of basic colloidal GDF-5 formulations for use in cosmetic compositions

Suitable GDF-5 formulations can be prepared by using different colloidal drug carriers. Basic compositions comprising colloidal carriers might i.e. comprise polymeric nanoparticles and lipid microparticles which are preparable as follows:

### A. Polymeric nanoparticles

Multiple polymeric nanoparticles were tested as colloidal drug carriers for GDF-5, i.e. resomers® (Boehringer Ingelheim, Germany) with different compositions and molecular weights: PLA resomers® R202H and R202S (poly-(DL)-lactide), PLGA resomers® RG502, RG502H, RG503H and RG504H (poly-(DL-lactide-co-glycolide)). As general acidic solvent for GDF-5 10 mM HCl was used, investigated emulsifying agents were polyvinyl alcohol (PVA), poloxamer188 (P188) and polyvinylpyrrolidone (PVP). Synthesis of nanoparticles was performed with the double-emulsion method under sterile conditions according to FIG. 17 (i.e. w1-phase = 500 µg protein in 142.1 µl 10 mM HCl, w2-phase = 160 mg emulsifier in 30 ml water, o-phase = 40 mg polymer in 3.1 ml methylenchloride). Emulsions were created by sonication for 40 sec (w1/0) and 2 min (w1/o/w2), respectively. After removal of the organic solvent by vaporization, precipitation of the polymer and subsequent encapsulation of the protein occurred. Particle size was determined with the help of an electron microscope. Resomer® R202H particles (FIG. 18) combined with emulsifier polyvinyl alcohol or poloxamer188 gave the best results concerning particle morphology and protein load. Average size particles loaded with GDF-5 was approximately 320 nm, average drug concentration 66 µg per mg particle. Due to relatively low protein load, the tested organic nanoparticles were considered to be appopriate drug carriers for cosmetic compositions comprising GDF-5-related proteins since cosmetics contain comparatively low amounts of active ingredients.

### B. Lipid microparticle compositions

Especially suitable for dermal purposes are lipid formulations. Several lipid microparticle (LMP) compositions were created and tested as colloidal drug carriers for cosmetic compositions comprising GDF-5-related proteins.

### 1. LMP compositions comprising natural (plant) oils

For example, 22.93 µl olive oil (21.1 mg) was combined with an equal weight of phosphatidylserine into a 1.5 ml microcentrifuge tube. 956 µl of 50 mM succinate buffer, pH 4.25, was added to bring the total volume to 1 ml. The mixture was vortexed for 30 seconds and placed in an ice-ethanol bath to keep the temperature constantly at 4°C. The sample was sonicated for one hour with an average power output of 22 watts on a 25% duty cycle with a cycle period of 20 seconds. After 1 hour, the emulsion appeared transparent with an amber colour when held up to the light, indicating that the emulsion spheres were getting smaller and reached the expected diameter of approximately 50 nm (FIG. 19). At this point, the LMP mixture was transferred by pipette into a microcentrifuge tube containing 2.5 mg of the lyophilized GDF-5 protein. The sample was vortexed until the protein became solubilized. Introduction of the protein caused the mixture to become opaque, indicating a partial disruption of the LMP. The LMP mixture was then sonicated under the same conditions for an additional 45 minutes, restoring the previous transparency. At this point, the lipid microparticle mixture was vortexed on a low setting while 0.5 M NaOH was added in 10 µl increments until pH 7.0 was reached. The pH was measured by placing a small amount of solution on a pH strip. In some cases, the solutions were additionally filtered through a 0.2 µm filter. The protein content of the lipid microparticles loaded with GDF-5 was determined via bicinchoninic add (BCA) assay (Smith, P.K. et al. (1985), Anal. Biochem. 150, 76-85). GDF-5 concentration was of the expected order (around 2.5 mg/ml).

No precipitation was noted during the titration, indicating that the lipid microparticle composition was suitable to carrying high amounts of GDF-5 (up to 2.5 mg/ml) even at neutral pH. As a consequence, the tested LMPs were considered to be appropriate drug carriers for cosmetic compositions comprising GDF-5-related proteins.

### 2. LMP compositions comprising triglycerides

| | | |
|---|---|---|
| Composition example: | Myglyol® 812 | 0.95 g |
| | Phospholipon® 80 | 0.925 g |
| | Citrate buffer pH 4.25 | 6.7 ml |
| | GDF-5 (4.4 mg/ml) | 0.227 ml |
| | NaOH 0.5M | 1 ml |
| | Water | ad 10 ml |

Triglycerides (i.e. Myglyol® 812), emulsifiers (i.e. Phospholipon® 80) and citrate buffer were combined and vortexed for 30 sec at 3000 rounds per minute). The sample was sonicated for one hour with an average power output of 22 watts and continuous cooling in an ice bath. At this point, GDF-5 solution was added to the LMP mixture. The sample was then vortexed and sonicated under the same conditions for an additional hour. The lipid microparticle mixture was vortexed on a low setting while 0.5 M NaOH was added in 10 µl increments until pH 7.0 was reached.

### 3. LMP compositions without oily phase

| | | |
|---|---|---|
| Composition example: | Phospholipon® 80 | 0.925 g |
| | Citrate buffer pH 4.25 | 7.7 ml |
| | GDF-5 (4.4 mg/ml) | 0.227 ml |
| | NaOH 0.5M | 1 ml |
| | Water | ad 10 ml |

Manufacturing instructions: see LMP compositions comprising triglycerides

### C. Microemulsions

| | | |
|---|---|---|
| Composition example: | Glycerylisostearat | 0,05 g |
| | Phospholipon® 80 | 0.05 g |
| | Isoceteth-20 | 0.25 g |
| | Isopropylmyristat | 0,15 g |
| | Glycerol | 0.5 g |
| | Citrate buffer pH 4.25 | 7.7 ml |
| | GDF-5 (4.4 mg/ml) | 0.227 ml |
| | NaOH 0.5M | 1 ml |
| | Water | ad 10 ml |

A w/o (water dispersed in oil) microemulsion was created as follows: (Glycerylisostearat, Phospholipon® 80, Isoceteth-20, Isopropylmyristat) were combined and slowly stirred (300 rounds per minute) at 100° C to form a lipid phase. Water soluble compounds (Glycerol, citrate buffer and growth factor solution) were separately combined. After cooling down to room temperature, the water soluble mixture was added to the lipid phase in small portions. Homogeneous distribution was achieved by additional stirring.

### Example 5: Testing of colloidal GDF-5 formulations

The biological activity of colloidal GDF-5 formulations according to example 4 was tested by means of standard alkaline phosphatase assays as i.e. described in example 3. According to the test results, all chosen colloidal formulations (polymeric nanoparticles, lipid microparticles and microemulsions) featured considerable GDF-5-related bioactivity. In addition, integrity testing of encapsulated GDF-5 was checked by reverse phase HPLC and SDS-PAGE methods.

In order to compare the GDF-5 LMP formulations with a GDF-5 reference standard and to get information about possible aggregation or degradation, a common RP-HPLC analysis of GDF-5 LMP-formulations which separates molecules according to their polarity was used. The chromatography took place at a stationary phase (column: Vydac C18, 5 µm, Phase 218TP52, Protein and Peptide) and a mobile phase under high pressure. The elution of the protein and potential impurities during the chromatographie was achieved through a gradient of the mobile phase from polar (0.15 % trifluoroacetic acid in water with 35 % acetonitrile) to less polar (0.15 % trifluoroacetic acid in water with 100 % acetonitrile). For the RP-HPLC analysis, the samples were centrifuged for 10 minutes at 13000 g. The supernatants were diluted with 0.15 % TFA in 35 % acetonitrile. The samples were centrifuged again and the supernatants were applied to the RP-HPLC. No GDF-5 could be detected in the supernatant, indicating that GDF-5 was completely immobilized and enveloped by the lipids.

For detection of potential protein degradation, microparticle pellets with encapsulated GDF-5 were heated for 10 min at 80° C to solubilize the lipids, and were processed further via SDS-PAGE. Protein size separation (see FIG. 22) showed only very few degradation products and integrity of most of the GDF-5 protein.

Finally, successful penetration testing was done to evaluate the dermal absorption of the GDF-containing compositions of the invention. Various techniques for the assessment of dermal absorption and percutaneous penetration have been developed. For ethical reasons, such testing should be done with excised skin samples by means of established *in vitro* assays. The following *in vitro* standard protocol for dermal absorption and percutaneous penetration of compositions designed for single or repetitive topical administration has been developed from W. Diembeck and H.-J. Duesing from Beiersdorf AG, Germany. This and other assay systems which utilize the principles outlined in the "OECD new guideline proposal on in vitro percutaneous apsorption of chemicals" are also described in Diembeck et al. 1999: Test guidelines for in vitro assessment of dermal absorption and percutaneous penetration of cosmetic ingredients. Food and Chemical Technology 37, 191-205.

The experiments are carried out with unboiled back skin of selected female pigs (about 130 days old and 100 kg in weight). The fresh back skin (about 30 x 40 cm) is cut in strips of about 10 x 40 cm. Most of the subcutaneous fat layer is removed and skin is rinsed with warm tap water. A circular punch with 5 cm inner diameter is used to cut skin discs which fit into the penetration cells. The discs are put into evacuated plastic bags and stored at -20° C until use. Test samples are blended with radiolabelled test substances and mixed thoroughly. Sufficiently labelled material should give a specific activity of about 0.4 Mbq/mg. Thawed skin discs are dabbed dry with cotton wool.

The skin is mounted on the receptor compartment of a penetration cell (see FIG. 23) with epidermal side up and the donor chamber clamped in place. The test sample is applied onto the epidermal skin side and evenly distributed within the circular application area (2.5 cm diameter) of the penetration cell. After 24 hours, the absorption/penetration experiment is terminated. Experiments are usually carried out in triplicate. Analytical samples are prepared as follows:

The upper penetration cell is placed into 15 ml of a suitable solvent which is capable of solubilizing the test substance out of the emulsion and removing residues sticking to the glass. An aliquot of 1 ml is added to 10 ml scintillation cocktail for radioanalysis. After removal of the skin disc from the lower penetration cell, ca 5 ml receptor fluid is pipetted into a flask. The bottom side of the skin disk and the lower penetration cell are washed each with 1 ml distilled water which are then added to the receptor fluid. The flask is filled up to 10 ml with distilled water. A 1 ml aliquot is added to 10 ml scintillation cocktail for radioanalysis. Excessive test sample is removed from the skin surface and transferred into a scintillation vial.

Homy layer (stratum corneum) is stripped from the treated skin with adhesive tape (Tesafilm Type 4129, width 25 mmm, Beiersdorf, Germany). The strips are placed in scintillation vials. Epidermis and dermis are heat separated by placing the skin samples, epidermal side down, under light pressure on a hotplate at 80° C for 45 seconds. The epidermis is peeled from the dermis with a spatula and solubilized in 2 ml soluene (Packard). After cooling to ambient temperature, 10 ml scintillation cocktail are added to the epidermis solution. The outer, hard edge of the dermis, which does not contain test substance, is cut off and discarded. The remaining dermis is cut into pieces and dissolved in 12 ml soluene (Packard) for 24 hours. After cooling to ambient temperature, 10 ml scintillation cocktail are added to 1 ml dermis solution.

Radioactivity of all samples is measured with a liquid scintillation counter type LSC3801 (Beckman Instruments). The radioactivity in all samples is used to calculate the total non-absorbed (skin surface and upper cell surface) and absorbed radioactivity (horny layer, epidermis, dermis, receptor fluid). The sum of these values (total recovered radioactivity) was normalized to 100%. The percentages of radioactivity in surface, horny layer etc. were recalculated as percentages of the normalized total radioactivity to yield information about the distribution of the applied test substance (non-absorbed v. absorbed). The sum of the normalized, absorbed radioactivity was set to 100% and the horny layer, epidermis, dermis and receptor fluid data were recalculated as percentages of the normalized, absorbed radioactivity to yield information about the test substance distribution within the skin.

Additional successful permeation experiments were performed by using heat separated human skin specimens from one skin donor in static Franz diffusion cells (3 cm² skin surface). 300 µL of the topical formulations (solutions) were applied. Samples were taken at 3 time points during a 48 h study period. All assays were performed in triplicate per formulation.

## Claims

1. A cosmetic, non-therapeutic method for the prevention, deceleration or reversal of dermal aging processes selected from the group consisting of chronological aging and photoaging, wherein the mammalian extracellular matrix (ECM) composition is altered by use of one or more GDF-5-related proteins and/or nucleic acid(s) encoding said protein(s), wherein GDF-5 related protein is any naturally occurring or artificially created protein which comprises a cysteine-knot-domain with an amino acid identity of at least 60% to the 102 aa cysteine-knot-domain of human GDF-5 (amino acids 400-501 of SEQ ID NO: 1).

2. The method of claim 1, wherein the ECM content of a substance selected from the group consisting of collagen type I, collagen type III, collagen type IV, collagen type VII, fibrillin-1 and -2, hyaluronic acid, elastin and decorin is increased.

3. The method of any one of the previous claims, further **characterized in that** said method comprises topical, transdermal or subcutaneous administration of a composition containing one or more GDF-5-related proteins and/or nucleic acid(s) encoding said protein(s).

4. A cosmetic, non-therapeutic use of a composition comprising one or more GDF-5-related proteins and/or nucleic acid(s) encoding said protein(s), wherein GDF-5 related protein is any naturally occurring or artificially created protein which comprises a cysteine-knot-domain with an amino acid identity of at least 60% to the 102 aa cysteine-knot-domain of human GDF-5 (amino acids 400-501 of SEQ ID NO: 1) for the improvement and/or maintenance of dermal appearance, selected from
a) amelioration or prevention of wrinkle,
b) retardation of dermal aging,
c) thickening of basement membrane, and
d) reduction of epidermal thickness.

5. The use according to claim 4, wherein the concentration of said GDF-5-related protein in the composition is between 0.1 µg/ml and 10 µg/ml.

6. The use according to any one of claims 4 and 5, wherein said GDF-5-related protein is a mammalian GDF-5 protein and/or a variant thereof, wherein said variant means biologically active fragment of a GDF-5 protein, biologically active protein construct which contains additional sequences in excess to the original sequence of the GDF-5 protein or any combination thereof.

7. The use according to any one of claims 4 to 6, wherein said composition comprises mature human GDF-5 or recombinant mature human GDF-5 (amino acids 1-120 or 2-120 of SEQ ID NO:3) or recombinant monomeric human GDF-5 (amino acids 1-120 or 2-120 of SEQ ID NO:4).

8. The use according to any one of the claims 4 to 7, wherein the composition comprises a colloidal drug carrier.

9. The use according to claim 8 wherein said composition comprises polymeric nanoparticles and/or lipid microparticles.

10. The use according to any one of claims 4 to 9, wherein said composition is administered topically, transdermally or subcutaneously.

11. The use according to any one of claims 4 to 10, wherein said composition is administered in a form selected from the group consisting of a lotion, a gel, a cream, an ointment, an aerosol spray, a lipstick, a plaster, a facial pack, a bathing agent, a facial cleansing agent, a bath detergent, a bathing soap, a sun cream, a sun lotion, an after sun lotion, a self tanning lotion, a microemulsion, an oil-in-water emulsion, a water-in-oil emulsion, a w/o/w emulsion, an o/w/o emulsion or a milky lotion.

12. The use according to any one of claims 4 to 11, wherein said composition further comprises at least one ingredient selected from the group consisting of hyaluronic acid, a retinoid, an antioxidant, an alpha-hydroxy acid, a peptide, a growth factor, a preservative, a fragrance, an oil, an alcohol, a fatty ester, an UV filter substance and a vitamin.

## Patentansprüche

1. Ein kosmetisches, nicht-therapeutisches Verfahren zur Vorbeugung, Verzögerung oder Umkehrung von Hautalterungsprozessen ausgewählt aus der Gruppe bestehend aus zeitlicher Alterung und lichtbedingter Alterung, wobei die Säugetier extrazelluläre Matrix (EZM) Zusammensetzung verändert wird durch die Verwendung von ein oder mehreren GDF-5-verwandten Proteinen und/oder Nukleinsäure(n), welche diese(s) Protein(e) kodieren, wobei GDF-5-verwandtes Protein irgendein natürlich vorkommendes oder künstlich hergestelltes Protein ist, welches eine Cystein-Knoten-Domäne mit einer Aminosäureidentität von mindestens 60% zu der 102 AS Cystein-Knoten-Domäne des humanen GDF-5 (Aminosäuren 400-501 der SEQ ID NR: 1) umfasst.

2. Verfahren nach Anspruch 1, wobei der EZM-Gehalt einer Substanz ausgewählt aus der Gruppe bestehend aus Kollagen Typ I, Kollagen Typ III, Kollagen Typ IV, Kollagen Typ VII, Fibrillin-1 und -2, Hyaluronsäure, Elastin und Dekorin erhöht ist.

3. Verfahren nach einem der vorhergehenden Ansprüche, weiter **gekennzeichnet dadurch, dass** dieses Verfahren topische, transdermale oder subkutane Verabreichung einer Zusammensetzung, welche ein oder mehrere GDF-5-verwandte Proteine und/oder Nukleinsäure(n), welche diese(s) Protein kodiert, enthält.

4. Kosmetische, nicht-therapeutische Verwendung einer Zusammensetzung umfassend ein oder mehrere GDF-5-verwandte Proteine und/oder Nukleinsäure(n), welche diese(s) Protein(e) kodieren, wobei GDF-5-verwandtes Protein irgendein natürlich vorkommendes oder künstlich hergestelltes Protein ist, welches eine Cystein-Knoten-Domäne mit einer Aminosäureidentität von mindestens 60% zu der 102 AS Cystein-Knoten-Domäne des humanen GDF-5 (Aminosäuren 400-501 der SEQ ID NR: 1) für die Verbesserung und/oder Aufrechterhaltung des Aussehens der Haut umfasst, ausgewählt aus
a) Verbesserung oder Vorbeugung von Falten,
b) Verzögerung der Hautalterung,
c) Verdickung der Basalmembran, und
d) Verringerung der epidermalen Dicke.

5. Verwendung nach Anspruch 4, wobei die Konzentration des GDF-5-verwandten Proteins in der Zusammensetzung zwischen 0,1 µg/ml und 10µg/ml ist.

6. Verwendung nach einem der Ansprüche 4 und 5, wobei das GDF-5-verwandte Protein ein Säugetier GDF-5 Protein und/oder eine Variante davon ist, wobei diese Variante ein biologisch aktives Fragment eines GDF-5 Proteins, ein biologisch aktives Protein Konstrukt, welches zusätzlich Sequenzen im Überschuss zu der original Sequenz des GDF-5 Proteins enthält, oder irgendeinen Kombination davon, bedeutet.

7. Verwendung nach einem der Ansprüche 4 bis 6, wobei die Zusammensetzung ein reifes, humanes GDF-5 oder rekombinantes, reifes, humanes GDF-5 (Aminosäuren 1-120 oder 2-120 der SEQ ID NR: 3) oder rekominantes, monomeres humanes GDF-5 (Aminosäuren 1-120 oder 2-120 der SEQ ID NR: 4) umfasst.

8. Verwendung nach einem der Ansprüche 4 bis 7, wobei die Zusammensetzung einen kolloidalen Arzneimittelträger umfasst.

9. Verwendung nach Anspruch 8, wobei diese Zusammensetzung polymere Nanopartikel und/oder Lipidmikropartikel umfasst.

10. Verwendung nach einem der Ansprüche 4 bis 9, wobei diese Zusammensetzung topisch, transdermal oder subkutan verabreicht wird.

11. Verwendung nach einem der Ansprüche 4 bis 10, wobei diese Zusammensetzung in einer Form ausgewählt aus der Gruppe bestehend aus einer Lotion, einem Gel, einer Creme, einer Salbe, einem Aerosolspray, einem Lippenstift, einem Pflaster, einer Gesichtspackung, einem Bade-Wirkstoff, einem Gesichtsreinigungswirkstoff, einem Bade-Tensid, einer Badeseife, einer Sonnencreme, einer Sonnenlotion, einer After-Sun-Lotion, einer selbst-bräunenden Lotion, einer Mikroemulsion, einer Öl-in-Wasser Emulsion, einer Wasser-in-Öl Emulsion, einer W/O/W-Emulsion, einer O/W/O-Emulsion oder einer milchigen Lotion verabreicht wird.

12. Verwendung nach einem der Ansprüche 4 bis 11, wobei die Zusammensetzung weiter mindestens einen Inhaltsstoff ausgewählt aus der Gruppe bestehend aus Hyaluronsäure, einem Retinoid, einem Antioxidanz, einer Alphahydroxysäure, einem Peptid, einem Wachstumsfaktor, einem Konservierungsmittel, einem Duftstoff, einem Öl, einem Alkohol, einem Fettsäureester, einer UV-Filter Substanz und einem Vitamin enthält.

## Revendications

1. Procédé cosmétique non thérapeutique pour la prévention, la décélération ou l'inversion de processus de vieillissement dermique choisis dans le groupe consistant en le vieillissement chronologique et le photovieillissement, où la composition de la matrice extracellulaire (MEC) de mammifère est modifiée par l'utilisation d'une ou plusieurs protéines apparentées à GDF-5 et/ou d'un ou plusieurs acides nucléiques codant ladite ou lesdites protéines, où la protéine apparentée à GDF-5 est toute protéine naturelle ou créée artificiellement qui comprend un domaine à noeud de cystéines ayant une identité d'aminoacides d'au moins 60 % avec le domaine à noeud de cystéines de 102 aa de GDF-5 humaine (aminoacides 400-501 de SEQ ID NO : 1).

2. Procédé selon la revendication 1 où la teneur dans la MEC d'une substance choisie dans le groupe consistant en le collagène de type I, le collagène de type III, le collagène de type IV, le collagène de type VU, la fibrilline-1 et -2, l'acide hyaluronique, l'élastine et la décorine est abaissée.

3. Procédé selon l'une quelconque des revendications précédentes **caractérisé en outre en ce que** ledit procédé comprend l'administration topique, transdermique ou sous-cutanée d'une composition contenant une ou plusieurs protéines apparentées à GDF-5 et/ou un ou plusieurs acides nucléiques codant ladite ou lesdites protéines.

4. Utilisation cosmétique non thérapeutique d'une composition comprenant une ou plusieurs protéines apparentées à GDF-5 et/ou un ou plusieurs acides nucléiques codant ladite ou lesdites protéines, où la protéine apparentée à GDF-5 est toute protéine naturelle ou créée artificiellement qui comprend un domaine à noeud de cystéines ayant une identité d'aminoacides d'au moins 60 % avec le domaine à noeud de cystéines de 102 aa de GDF-5 humaine (aminoacides 400-501 de SEQ ID NO : 1) pour l'amélioration et/ou le maintien de l'aspect dermique, choisie parmi
a) l'amélioration ou la prévention des rides,
b) le retard du vieillissement dermique,
c) l'épaississement de la membrane basale, et
d) la réduction de l'épaisseur épidermique.

5. Utilisation selon la revendication 4 où la concentration de ladite protéine apparentée à GDF-5 dans la composition est entre 0,1 pg/ml et 10 µg/ml.

6. Utilisation selon l'une quelconque des revendications 4 et 5 où ladite protéine apparentée à GDF-5 est une protéine GDF-5 de mammifère et/ou une variante de celle-ci, où ladite variante signifie un fragment biologiquement actif d'une protéine GDF-5, une construction de protéine biologiquement active qui contient des séquences supplémentaires en plus de la séquence originelle de la protéine GDF-5 ou toute combinaison de ceux-ci.

7. Utilisation selon l'une quelconque des revendications 4 à 6 où ladite composition comprend GDF-5 humaine mature ou GDF-5 humaine mature recombinante (aminoacides 1-120 ou 2-120 de SEQ ID NO: 3) ou GDF-5 humaine monomère recombinante (aminoacides 1-120 ou 2-120 de SEQ ID NO : 4).

8. Utilisation selon l'une quelconque des revendications 4 à 7 où la composition comprend un vecteur de médicament colloïdal.

9. Utilisation selon la revendication 8 où ladite composition comprend des nanoparticules polymères et/ou des microparticules lipidiques.

10. Utilisation selon l'une quelconque des revendications 4 à 9 où ladite composition est administrée par voie topique, transdermique ou sous-cutanée.

11. Utilisation selon l'une quelconque des revendications 4 à 10 où ladite composition est administrée sous une forme choisie dans le groupe consistant en une lotion, un gel, une crème, une pommade, un spray d'aérosol, un crayon à lèvres, un emplâtre, une présentation faciale, un agent pour le bain, un agent de nettoyage facial, un détergent pour le bain, un savon pour le bain, une crème solaire, une lotion solaire, une lotion après solaire, une lotion d'autobronzage, une microémulsion, une émulsion huile dans eau, une émulsion eau dans huile, une émulsion e/h/e, une émulsion h/e/h ou une lotion laiteuse.

12. Utilisation selon l'une quelconque des revendications 4 à 11 où ladite composition comprend en outre au moins un ingrédient choisi dans le groupe consistant en l'acide hyaluronique, un rétinoïde, un antioxydant, un alphahydroxyacide, un peptide, un facteur de croissance, un conservateur, un parfum, une huile, un alcool, un ester gras, une substance formant filtre UV et une vitamine.
